# EUROPEAN PATENT APPLICATION

(11) **EP 2 322 620 A2**
(43) Date of publication of application: **18.05.2011**
(21) Application number: 10196976.4
(22) Date of filing: 09.07.2008
(51) Int. Cl.: C12N 15/12, C07K 14/575, A61K 38/18

(54) **Analogs of human pancreatic polypeptide and their effects on feeding behaviour**

(30) Priority: 09.07.2007 GB 0713279; 28.04.2008 GB 0807720
(62) Divisional of application: 08775889.2
(71) Applicant: Imperial Innovations Ltd., South Kensington, London, SW7 2PG (GB)
(72) Inventor: Bloom, Stephen, London Greater London W12 0NN (GB)
(74) Representative: Hewson, Timothy John

(57) **Abstract**

Analogues of human pancreatic polypeptide, pharmaceutical compositions comprising said analogues wherein said analogues are useful in reducing appetite in a subject, reducing food intake in a subject or reducing calorie intake in a subject; and for the treatment of obesity or diabetes.

## Description

### 1. FIELD OF THE INVENTION

This application relates to the use of agents to control appetite, feeding, food intake, energy expenditure and calorie intake, particularly in the field of obesity.

### 2. BACKGROUND OF THE INVENTION

According to the National Health and Nutrition Examination Survey (NHANES III, 1988 8 to 1994), between one third and one half of men and women in the United States are overweight. In the United States, sixty percent of men and fifty-one percent of women, of the age of 20 or older, are either overweight or obese. In addition, a large percentage of children in the United States are overweight or obese.

The cause of obesity is complex and multi-factorial. Increasing evidence suggests that obesity is not a simple problem of self-control but is a complex disorder involving appetite regulation and energy metabolism. In addition, obesity is associated with a variety of conditions associated with increased morbidity and mortality in a population. Although the etiology of obesity is not definitively established, genetic, metabolic, biochemical, cultural and psychosocial factors are believed to contribute. In general, obesity has been described as a condition in which excess body fat puts an individual at a health risk.

There is strong evidence that obesity is associated with increased morbidity and mortality. Disease risk, such as cardiovascular disease risk and type 2 diabetes disease risk, increases independently with increased body mass index (BMI). Indeed, this risk has been quantified as a five percent increase in the risk of cardiac disease for females, and a seven percent increase in the risk of cardiac disease for males, for each point of a BMI greater than 24.9 (see Kenchaiah et al., N. Engl. J. Med. 347:305, 2002; Massie, N Engl. J. Med. 347:358, 2002). In addition, there is substantial evidence that weight loss in obese persons reduces important disease risk factors. Even a small weight loss, such as 10% of the initial body weight in both overweight and obese adults has been associated with a decrease in risk factors such as hypertension, hyperlipidemia, and hyperglycemia.

Although diet and exercise provide a simple process to decrease weight gain, overweight and obese individuals often cannot sufficiently control these factors to effectively lose weight. Pharmacotherapy is available; several weight loss drugs have been approved by the Food and Drug Administration that can be used as part of a comprehensive weight loss program. However, many of these drugs have serious adverse side effects. When less invasive methods have failed, and the patient is at high risk for obesity related morbidity or mortality, weight loss surgery is an option in carefully selected patients with clinically severe obesity. However, these treatments are high-risk, and suitable for use in only a limited number of patients. It is not only obese subjects who wish to lose weight. People with weight within the recommended range, for example, in the upper part of the recommended range, may wish to reduce their weight, to bring it closer to the ideal weight. Thus, a need remains for agents that can be used to effect weight loss in overweight and obese subjects.

Pancreatic Polypeptide (PP) is a gut hormone that is released from the pancreas in response to ingestion of food. PP is believed to act primarily at Y4 receptors. Plasma PP has been shown to be reduced in conditions associated with increased food intake and elevated in anorexia nervosa. Peripheral administration of mouse PP to mice was found by Asakawa *et al.* (Asakawa et al., Gastroenterology, 2003, 124, 1325-1336 and Asalcawa et al., Peptides, 1999, 20, 1445-1448) to induce a negative energy balance by decreasing food intake and stimulating energy expenditure. In man, short term administration of human PP to children with Prader-Willi syndrome *(Zipf et al.,* 1990, **51**, 162-166) at a rate of 100 pmol/kg/h for 90 minutes was found to have no effect on appetite. More recently, a somewhat larger infusion of human PP (10 pmol/kg/min (ie 600pmol/kg/h) for 90 minutes) was found to reduce appetite and food intake in healthy volunteers *(*Batterham, et al, J Clin. Endrocrinol. Metab., 2003, 88(8), 3989-3992). The infusion caused a decrease in 24 hour cumulative energy intake of 25%. At a lower infusion rate of 5 pmol/kg/min (ie 300 pmol/kg/h) human PP for 90 minutes, a reduction in energy intake of 11 % was observed in healthy human volunteers (Jesudason, et al., Br. J. Nutrition, 2007, 97, 426-429). It is clear, however, that the activity of native PP, and its rate of degradation in the blood are too low and too rapid respectively for the compound to represent a viable long-term treatment in humans. Accordingly, there remains a need for analogues of pancreatic polypeptide with improved properties.

Some analogues of Pancreatic Polypeptides are known. Cabrele *et* a/.synthesised various chimeras of Pancreatic Polypeptide and Neuropeptide Y (Cabrele et al., Peptides, 2001, 22, 365-378). The chimeras were found to have strong affinity for the Y5 receptor. The analogues, 2-36[K⁴, RYYSA¹⁹⁻²³]pp, [K4]pp and [RYYSA¹⁹⁻²³]PP had strong affinity to the Y5-receptor, and they were was found to have a strong stimulatory effect on food intake when injected intracerebroventricularly (McCrea *et al.,* 2000, **87**, 47-58).

In WO2005/089786 there are described Y4 selective receptor agonists that are derivatives of various gut hormones including PP. In WO2005/089789 and W02007/038943 there are described Y2 selective receptor agonists that are derivatives of various gut hormones including PP. The agonists therein include some derivatives of PP with the Glu at position 4 replaced by Lys. In WO2005/089790 there are described Y2 and Y4 selective receptor agonists that are derivatives of various gut hormones including PP. The agonists therein include some derivatives of PP with the Pro at position 34 replaced by Gln. In WO2006/091506 there are described various N-terminal modified derivatives of PP.

### 3. SUMMARY OF THE INVENTION

The present invention provides an analogue of human Pancreatic Polypeptide (SEQ. ID No 1) which differs from native human pancreatic polypeptide in one of more of the following respects:

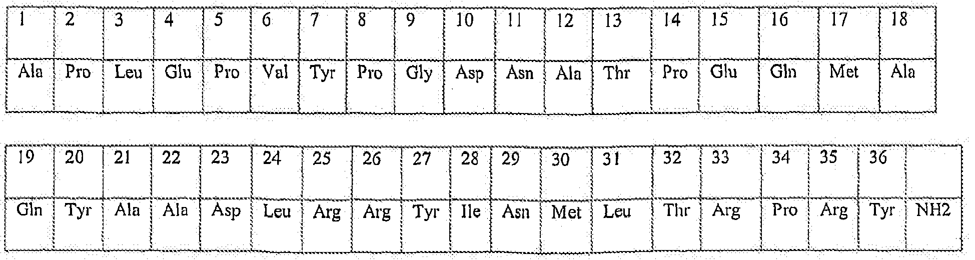

a) it has the Ala at position 1
(i) replaced by an alternative amino acid selected from D-Ala, Asp, Glu, Gly, His, Tyr, Lys, acylated Ala and acylated Lys, the acyl group being selected from: CO-C₁₋₂₀ alkyl, CO-C₂₋₂₀ alkenyl, CO-C₅₋₁₀ aryl and CO-C₅-₁₀ ar-C₁-₂₀ alkyl, and/or
(ii) furnished with an additional amino acid at position 0, selected from Gly, Lys, His, Glu, Asp, Pro, Ile, Phe, Val, Cys, Tyr, TyrTyrTyr, Ala, D-Ala, and acylated Ala, the acyl group being selected from: CO-C₁₋₂₀ alkyl, CO-C₂-₂₀ alkenyl, CO-C₅-10 aryl and CO-C₅₋₁₀ar-C₁₋₂₀ alkyl; or
(iii) absent
b) at positions 3, 4, 5, 6, 7 and 10, it has
(i) the Leu at position 3 replaced by the alternative amino acid Ile or Ser; and/or
(ii) the Glu at position 4 replaced by the alternative amino acid Lys; and/or
(iii) the Pro at position 5 replaced by the alternative amino acid Ala; and/or
(iv) the Val at position 6 replaced by an alternative amino acid selected from Glu, His, Ile, Leu, Ser, Phe, Cys, Thr, Ala, Arg, Asp, Lys, Tyr and Met; and/or
(v) the Tyr at position 7 replaced by the alternative amino acid Ala, Asn or Phe; and/or
(vi) the Asp at position 10 replaced by the alternative amino acid Glu;
c) it has the Asn at position 11 replaced by an alternative amino acid selected from Asp and Tyr; and/or the Thr at position 13 replaced by the alternative amino acid Ser;
d) at positions 14 to 26, it has one or more of the native amino acids 14 to 26 (first row, bold) replaced with an amino acid selected from the amino acids in the respective column below it:

| 14 | 15 | 16 | 17 | 18 | 19 | 20 | 21 | 22 | 23 | 24 | 25 | 26 |
|---|---|---|---|---|---|---|---|---|---|---|---|---|
| **Pro** | **Glu** | **Gln** | **Met** | **Ala** | **Gln** | **Tyr** | **Ala** | **Ala** | **Asp** | **Leu** | **Arg** | **Arg** |
| Glu | Gln | Glu | Leu | Asn | Arg | | Tyr | Ser | Ala | Met | Lys | His |
| | | Asp | NLeu | Val | Lys | | Phe | Ile | Glu | Cys | NLeu | Lys |
| | | Lys-Acyl* | Ile | Ser | His | | Glu | Val | Ser | NLeu | | |
| | | | Arg | | Glu | | | Thr | Gly | | | |
| | | | Thr | | | | | | Gln | | | |
| | | | Gln | | | | | | Ile | | | |
| | | | Glu | | | | | | Leu | | | |
| | | | Lys | | | | | | NLeu | | | |
| | | | Val | | | | | | Val | | | |

| | | | | | | | | | | | | |
|---|---|---|---|---|---|---|---|---|---|---|---|---|
| * the acyl group being selected from: CO-C₁₋₂₀alkyl, CO-C₂-₂₀ alkenyl, CO-C₅-₁₀ aryl and CO-C₅₋₁₀ ar-C₁₋₂₀ alkyl | | | | | | | | | | | | |

e) at positions 29, 30 and 31, it has:
(i) the Met at position 30 replaced by an alternative amino acid selected from Leu, Arg, Glu, His, NLeu, Ile, Val, Phe, Thr, Asn, Cys and Lys; and/or
(ii) the Leu at position 31 replaced by an alternative amino acid selected from Ile and Val; and/or
(iii) the Asn at position 29 replaced by Asp
f) at positions 34, 35 and 36, it has:
(i) the Pro at position 34 replaced by the alternative amino acid Gln, Asn, DPro, Leu, His or NVa (Nor-valine); and/or
(ii) the Arg at position 35 replaced by the alternative amino acid Gln or HArg; and/or
(iii) the Tyr at position 36 replaced by the alternative amino acid Phe
g) it is dimerised by disulphide bond formation between a pair of Cys residues added to the two units at the N termini or as replacements for Leu at position 31 in the two units,
h) it is cyclised by disulphide bond formation between a pair of Cys residues added as a replacement amino acid residue at a pair of positions selected from the following list: 8 & 17, 8 & 20, 5 & 24, 2 & 27, or between a Cys residue added at a replacement amino acid at position 28 or 29 and a Cys residue added as an additional amino acid at position 0; whereby when the analogue has Ala1 absent then it has at least one further change other than one or more selected from Nle 17 and Nle30,
whereby when the analogue has one or more of N1e17, N1e30 and His34, then it has at least one further change other than the one or more selected from Nle17, Nle30 and His34, and
whereby when the analogue has Gln at position 34, then it has at least one further change other than one or more selected from Ile31, Va131, Leu30 and NLeu30.
for use (i) in the treatment (including prophylactic treatment) of obesity or diabetes in a subject, or (ii) in the reduction of appetite in a subject, the reduction of food intake in a subject, or in the reduction of calorie intake in a subject.

The invention also provides further uses of a compound of formula (I), methods that use a compound of formula (I), compositions comprising a compound of formula (I), and methods of making a compound of formula (I).

### 4. BRIEF DESCRIPTION OF THE FIGURES

Figure 1 shows the certain data pertaining to the examples
Figure 2 shows the data from an appetite suppression experiment carried out using three Example analogues of the invention as a bar chart.
Figure 3 shows the data from an appetite suppression experiment carried out using three Example analogues of the invention as a line graph.

### 5. SEQUENCE LISTING

The amino acid sequences listed in the application are shown using standard letter abbreviations for amino acids.

### 6. DEFINITIONS

In order to facilitate review of the various embodiments of this disclosure, the following explanations of specific terms are provided:
**Animal:** Living multi-cellular vertebrate organisms, a category that includes, for example, mammals and birds. The term mammal includes both human and non-human mammals. Similarly, the term "subject" includes both human and veterinary subjects.
**Appetite:** A natural desire, or longing for food. In one embodiment, appetite is measured by a survey to assess the desire for food. Increased appetite generally leads to increased feeding behavior.
**Appetite Suppressants:** Compounds that decrease the desire for food. Commercially available appetite suppressants include, but are not limited to, amfepramone (diethylpropion), phentermine, mazindol and phenylpropanolamine fenfluramine, dexfenfluramine, and fluoxetine.
**Body Mass Index (BMI):** A mathematical formula for measuring body mass, also sometimes called Quetelet's Index. BMI is calculated by dividing weight (in kg) by height² (in meters²). The current standards for both men and women accepted as "normal" are a BMI of 20-24.9 kg/m². In one embodiment, a BMI of greater than 25 kg/m² can be used to identify an obese subject. Grade I obesity corresponds to a BMI of 25-29.9 kg/m². Grade II obesity corresponds to a BMI of 30-40 kg/m²; and Grade III obesity corresponds to a BMI greater than 40 kg/m² (Jequier; Am. J Clin. Nutr. 45:1035-47, 1987), Ideal body weight will vary among species and individuals based on height, body build, bone structure, and sex.
**Conservative substitutions:** The replacement of an amino acid residue by another, biologically similar residue in a polypeptide. The term "conservative variation" also includes the use of a substituted amino acid, i.e. an amino with one or more atoms replaced with another atom or group, in place of a parent amino acid provided that antibodies raised to the substituted polypeptide also immunoreact with the unsubstituted polypeptide.
**Diabetes:** A failure of cells to transport endogenous glucose across their membranes either because of an endogenous deficiency of insulin and/or a defect in insulin sensitivity. Diabetes is a chronic syndrome of impaired carbohydrate, protein, and fat metabolism owing to insufficient secretion of insulin or to target tissue insulin resistance. It occurs in two major forms: insulin-dependent diabetes mellitus (IDDM, type I) and non-insulin dependent diabetes mellitus (NIDDM, type II) which differ in etiology, pathology, genetics, age of onset, and treatment.

The two major forms of diabetes are both characterized by an inability to deliver insulin in an amount and with the precise timing that is needed for control of glucose homeostasis. Diabetes type I, or insulin dependent diabetes mellitus (IDDM) is caused by the destruction of β cells, which results in insufficient levels of endogenous insulin. Diabetes type II, or non-insulin dependent diabetes, results from a defect in both the body's s sensitivity to insulin, and a relative deficiency in insulin production.

**Food intake:** The amount of food consumed by an individual. Food intake can be measured by volume or by weight. For example, food intake may be the total amount of food consumed by an individual. Or, food intake may be the amount of proteins, fat, carbohydrates, cholesterol, vitamins, minerals, or any other food component, of the individual. "Protein intake" refers to the amount of protein consumed by an individual. Similarly, "fat intake," "carbohydrate intake," "cholesterol intake," "vitamin intake," and "mineral intake" refer to the amount of proteins, fat, carbohydrates, cholesterol, vitamins, or minerals consumed by an individual.

**Hyperpolarization:** A decrease in the membrane potential of a cell. Inhibitory neurotransmitters inhibit the transmission of nerve impulses via hyperpolarization. This hyperpolarization is called an inhibitory postsynaptic potential (IPSP). Although the threshold voltage of the cell is unchanged, a hyperpolarized cell requires a stronger excitatory stimulus to reach threshold.

**Normal Daily Diet:** The average food intake for an individual of a given species. A normal daily diet can be expressed in terms of caloric intake, protein intake, carbohydrate intake, and/or fat intake. A normal daily diet in humans generally comprises the following: about 2,000, about 2,400, or about 2,800 to significantly more calories. In addition, a normal daily diet in humans generally includes about 12 g to about 45 g of protein, about 120 g to about 610 g of carbohydrate, and about 11 g to about 90 g of fat. A low calorie diet would be no more than about 85%, and preferably no more than about 70%, of the normal caloric intake of a human individual.

In animals, the caloric and nutrient requirements vary depending on the species and size of the animal. For example, in cats, the total caloric intake per pound, as well as the percent distribution of protein, carbohydrate and fat varies with the age of the cat and the reproductive state. A general guideline for cats, however, is 40 cal/lb/day (18.2 cal/kg/day). About 30% to about 40% should be protein, about 7% to about 10% should be from carbohydrate, and about 50% to about 62.5% should be derived from fat intake. One of skill in the art can readily identify the normal daily diet of an individual of any species.

**Obesity:** A condition in which excess body fat may put a person at health risk (see Barlow and Dietz, Pediatrics 102:E29, 1998; National Institutes of Health, National Heart, Lung, and Blood Institute (NHLBI), Obes. Res. 6 (suppl. 2):51S-209S, 1998). Excess body fat is a result of an imbalance of energy intake and energy expenditure. For example, the Body Mass Index (BMI) may be used to assess obesity. In one commonly used convention, a BMI of 25.0 kg/m² to 29.9 kg/m² is overweight, while a BMI of 30 kg/m² is obese.

In another convention, waist circumference is used to assess obesity. In this convention, in men a waist circumference of 102 cm or more is considered obese, while in women a waist circumference of 89 cm or more is considered obese. Strong evidence shows that obesity affects both the morbidity and mortality of individuals. For example, an obese individual is at increased risk for heart disease, non-insulin dependent (type 2) diabetes, hypertension, stroke, cancer (e.g. endometrial, breast, prostate, and colon cancer), dyslipidemia, gall bladder disease, sleep apnea, reduced fertility, and osteoarthritis, amongst others (see Lyznicki et al., Am. Fam. Phys. 63:2185,2001).

**Overweight:** An individual who weighs more than their ideal body weight. An overweight individual can be obese, but is not necessarily obese. For example, an overweight individual is any individual who desires to decrease their weight. In one convention, an overweight individual is an individual with a BMI of 25.0 kg/m² to 29.9 kg_{/}m²

**Pancreatic Polypeptide:** The term Pancreatic Polypeptide as used herein refers to a Panreatic Polypeptide, a hormone secreted into the blood by cells lining the lower small intestine (the ileum) and the colon. If no further limitation is placed on the term, it includes the peptide from any species.

**Pegylated and pegylation:** the process of reacting a poly(alkylene glycol), preferably an activated poly(alkylene glycol) to form a covalent bond. A facilitator may be used, for example an amino acid, e.g. lysine. Although "pegylation" is often carried out using poly(ethylene glycol) or derivatives thereof, such as methoxy poly(ethylene glycol), the term is not limited herein to the use of methoxy poly(ethylene glycol) but also includes the use of any other useful poly(alkylene glycol), for example poly(propylene glycol).

**Peripheral Administration:** Administration outside of the central nervous system. Peripheral administration does not include direct administration to the brain. Peripheral administration includes, but is not limited to intravascular, intramuscular, subcutaneous, inhalation, oral, rectal, transdermal or intra-nasal administration

**Polypeptide:** A polymer in which the monomers are amino acid residues which are joined together through amide bonds. When the amino acids are alpha-amino acids, either the L-optical isomer or the D-optical isomer can be used, the L-isomers being preferred. The terms "polypeptide" or "protein" as used herein encompass any amino acid sequence and include modified sequences such as glycoproteins. The term "polypeptide" is specifically covers naturally occurring proteins, as well as those which are recombinantly or synthetically produced. The term "polypeptide fragment" refers to a portion of a polypeptide, for example a fragment which exhibits at least one useful sequence in binding a receptor. The term "functional fragments of a polypeptide" refers to all fragments of a polypeptide that retain an activity of the polypeptide. Biologically functional peptides can also include fusion proteins, in which the peptide of interest has been fused to another peptide that does not decrease its desired activity.

**Therapeutically effective amount:** A dose sufficient to prevent advancement, or to cause regression of a disorder, or which is capable of relieving a sign or symptom of a disorder, or which is capable of achieving a desired result. In several embodiments, a therapeutically effective amount of a compound of the invention is an amount sufficient to inhibit or halt weight gain, or an amount sufficient to decrease appetite, or an amount sufficient to reduce caloric intake or food intake or increase energy expenditure.

### 7. DETAILED DESCRIPTION

As mentioned above, the invention provides an analogue (I) of human Pancreatic Polypeptide or a variant or derivative thereof; or a salt or solvate thereof; for use (i) in the treatment (including prophylactic treatment) of obesity or diabetes in a subject, or (ii) in the reduction of appetite in a subject, the reduction of food intake in a subject, or in the reduction of calorie intake in a subject.

It has been found that the analogues of the invention have improved properties over native human pancreatic polypeptide. The analogues have a longer half-life in the blood than native human pancreatic polypeptide and/or they have stronger binding to the human Y4 receptor than native human pancreatic polypeptide.

The half life of an analogue in the blood is important because increased duration of appetite suppression can be particularly important to avoid the effect known as "escape". A short duration appetite suppressant may reduce appetite for the time covered by one meal and, in that meal, the subject typically eats less food. If, however, the appetite suppressant is then metabolized or otherwise removed from the circulation of the subject, then by the time of the next mealtime, the subject can regain its "normal" appetite. In view of the subject having eaten a small meal at the previous mealtime, the subject may in fact have an increased appetite by the time of the second meal. If the subject satisfies that appetite, it is possible for the food intake over the two meals, in total, to be no lower than the food intake would have been without the appetite suppressant. That is to say that the subject may have "escaped" from the effects of the appetite suppressant. "Escape" can be reduced by using additional doses of appetite suppressant, or by using an appetite suppressant with a longer duration of action. If the subject has a reduced appetite for longer, then the degree to which it can make up the missed food from one meal in the next meal is reduced as there is a practical limit to the total capacity for food in a particular meal.

Furthermore, it has been found that prolonged continuous infusion of a short duration appetite suppressant is less effective than administration of a single dose of a longer acting appetite suppressant even if the level of the shorter acting suppressant is maintained in the bloodstream for a similar length of time as the longer acting suppressant by continuous dosing. Herein a compound is considered to have a longer half life in the blood than native human PP if it a) has an effect greater on appetite suppression than native human PP during the four hours from the end of hour 4 to the end of hour 8 after administration of the compound to a test animal (for example a mouse), or has a greater effect than native human PP during the first 24 hours after administration to a test animal (for example a mouse), or b) if its rate of tailing off of effectiveness in time is slower than that of native human PP, as assessed by comparing the ratio of food intake reduction (vs native human PP) between 0-4 hours and 0-1 hour (the "4:1 ratio") or between 0-24 hours and 0-1 hour (the "24:1 ratio") after administration to a test animal (for example a mouse).

The Y4 receptor is the primary receptor to pancreatic polypeptide (see Berglund et al. Experimental Biology and Medicine 2003, 228, 217-244). Binding to the Y4 receptor can be assessed by standard techniques. For example, an ELISA or fluorescence-based assay may be used to assess binding of a compound at the receptor. A cell-based functional assay can be used to assess whether a compound is an agonist or an antagonist of the receptor. Herein, a compound is considered to be a strong binder to the human Y4 receptor if it has a lower binding IC₅₀ than native human PP.

As mentioned above, the compounds of the invention differ from native human pancreatic polypeptide in one of more of the following respects:
a) it has the Ala at position 1
(i) replaced by an alternative amino acid selected from D-Ala, Asp, Glu, Gly, His, Tyr, Lys, acylated Ala and acylated Lys, the acyl group being selected from: CO-C₁₋₂₀ alkyl, CO-C₂-₂₀ alkenyl, CO-C₅₋₁₀ aryl and CO-C₅-₁₀ ar-C₁₋₂₀ alkyl, and/or
(ii) furnished with an additional amino acid at position 0, selected from Gly,Lys, His, Glu, Asp, Pro, Ile, Phe_{,} Val, Cys, Tyr, TyrTyrTyr, Ala, D-Ala, and acylated Ala, the acyl group being selected from: CO-C₁₋₂₀ alkyl, CO-C₂-₂₀ alkenyl, CO-C₅-10 aryl and CO-C₅₋₁₀ ar-C₁₋₂₀alkyl; or
(iii) absent
b) at positions 3, 4, 5, 6, 7 and 10, it has
(i) the Leu at position 3 replaced by the alternative amino acid Ile or Ser; and/or
(ii) the Glu at position 4 replaced by the alternative amino acid Lys; and/or
(iii) the Pro at position 5 replaced by the alternative amino acid Ala; and/or
(iv) the Val at position 6 replaced by an alternative amino acid selected from Glu, His, Ile, Leu, Ser, Phe, Cys, Thr, Ala, Arg, Asp, Lys, Tyr and Met; and/or
(v) the Tyr at position 7 replaced by the alternative amino acid Ala, Asn or Phe; and/or
(vi) the Asp at position 10 replaced by the alternative amino acid Glu;
c) it has the Asn at position 11 replaced by an alternative amino acid selected from Asp and Tyr; and/or the Thr at position 13 replaced by the alternative amino acid Ser;
d) at positions 14 to 26, it has one or more of the native amino acids 14 to 26 (first row, bold) replaced with an amino acid selected from the amino acids in the respective column below it:

| 14 | 15 | 16 | 17 | 18 | 19 | 20 | 21 | 22 | 23 | 24 | 25 | 26 |
|---|---|---|---|---|---|---|---|---|---|---|---|---|
| **Pro** | **Glu** | **Gln** | **Met** | **Ala** | **Gln** | **Tyr** | **Ala** | **Ala** | **Asp** | **Leu** | **Arg** | **Arg** |
| Glu | Gln | Glu | Leu | Asn | Arg | | Tyr | Ser | Ala | Met | Lys | His |
| | | Asp | NLeu | Val | Lys | | Phe | Ile | Glu | Cys | NLeu | Lys |
| | | Lys-Acyl* | Ile | Ser | His | | Glu | Val | Ser | NLeu | | |
| | | | Arg | | Glu | | | Thr | Gly | | | |
| | | | Thr | | | | | | Gln | | | |
| | | | Gln | | | | | | Ile | | | |
| | | | Glu | | | | | | Leu | | | |
| | | | Lys | | | | | | NLeu | | | |
| | | | Val | | | | | | Val | | | |

| | | | | | | | | | | | | |
|---|---|---|---|---|---|---|---|---|---|---|---|---|
| * the acyl group being selected from: CO-C₁₋₂₀ alkyl, CO-C₂-₂₀ alkenyl, CO-C₅-₁₀ aryl and CO-C₅₋₁₀ ar-C₁₋₂₀ alkyl | | | | | | | | | | | | |

e) at positions 29, 30 and 31, it has:
(i) the Met at position 30 replaced by an alternative amino acid selected from Leu, Arg, Glu, His, NLeu, Ile, Val, Phe, Thr, Asn, Cys and Lys; and/or
(ii) the Leu at position 31 replaced by an alternative amino acid selected from Ile and Val; and/or
(iii) the Asn at position 29 replaced by Asp
f) at positions 34, 35 and 36, it has:
(i) the Pro at position 34 replaced by the alternative amino acid Gln, Asn, DPro, Leu, His or NVa (Nor-valine); and/or
(ii) the Arg at position 35 replaced by the alternative amino acid Gln or HArg; and/or
(iii) the Tyr at position 36 replaced by the alternative amino acid Phe
g) it is dimerised by disulphide bond formation between a pair of Cys residues added to the two units at the N termini or as replacements for Leu at position 31 in the two units,
h) it is cyclised by disulphide bond formation between a pair of Cys residues added as a replacement amino acid residue at a pair of positions selected from the following list: 8 & 17, 8 & 20, 5 & 24, 2 & 27, or between a Cys residue added at a replacement amino acid at position 28 or 29 and a Cys residue added as an additional amino acid at position 0; whereby when the analogue has Ala1 absent then it has at least one further change other than one or more selected from Nle17 and Nle30,
whereby when the analogue has one or more of Nle17, Nle30 and His34, then it has at least one further change other than the one or more selected from Nle17, Nle30 and His34, and
whereby when the analogue has Gln at position 34, then it has at least one further change other than one or more selected from Ile31, Val31, Leu30 and NLeu30.
for use (i) in the treatment (including prophylactic treatment) of obesity or diabetes in a subject, or (ii) in the reduction of appetite in a subject, the reduction of food intake in a subject, or in the reduction of calorie intake in a subject.

In one embodiment, analogues which a) have the Ala at position 1
(i) replaced by an alternative amino acid selected from D-Ala, Lys, Asp, Glu, Gly, His, Tyr, Lys, acylated Ala and acylated Lys, the acyl group being selected from: CO-C₁₋₂₀ alkyl, CO-C₂-₂₀ alkenyl, CO-C₅-₁₀ aryl and CO-C₅₋₁₀ ar-C₁₋₂₀ alkyl, and/or
(ii) furnished with an additional amino acid at position 0, selected from Gly, Lys, His, Glu, Asp, Pro, Ile, Phe, Val, Cyc, Tyr, TyrTyrTyr, Ala, D-Ala, and acylated Ala, the acyl group being selected from: CO-C₁₋₂₀ alkyl, CO-C₂₋₂₀ alkenyl, CO-C₅₋. 10 aryl and CO-C₅₋₁₀ ar-C₁₋₂₀ alkyl; or
(iii) absent
are analogues in which the Ala at position 1 is (i) replaced by an alternative amino acid selected from D₋Ala, Lys, Asp, Glu, Gly, His, Tyr, Lys, acylated Ala and acylated Lys, the acyl group being selected from: CO-C₁₋₂₀ alkyl, CO-C₂₋₂₀ alkenyl, CO-C₅₋₁₀ aryl and CO-C₅₋₁₀ ar-C₁₋₂₀ alkyl. The analogues have a longer half-life in the blood than native human PP and/or they have stronger binding to the Y4 receptor than native human PP. For example, the compound of SEQ ID NO:11 (code 6435) with the Ala at position 1 replaced with lauryl-lysine is a stronger binder to the Y4 receptor than native human PP; similarly, the compounds of SEQ ID NO:72 (code 6674) and SEQ ID NO:73 (code 6675) are stronger binders than the compound of SEQ ID NO:72 (code 6672). For example, the compound of SEQ ID NO:228 (code 4055400) with the Ala at position 1 replaced with D-Ala has a longer half-life in the blood than native human PP.

Preferred replacement amino acids at position 1 are selected from Lys, D-Ala and Acyl-Lys where the acyl group is C0-C₁₋₂₀ alkyl or CO-C₂-₂₀ alkenyl, for example CO-C₁-₂₀ alkyl. Most preferred are Lys, D-Ala and Lauryl-Lys.

In an embodiment, analogues which a) have the Ala at position 1 altered as in a) above, have the Ala furnished with an additional amino acid at position 0, selected from Gly, Lys, His, Glu, Asp, Pro, Ile, Phe, Val, Cys, Tyr, TyrTyrTyr, Ala, D-Ala, and acylated Ala, the acyl group being selected from: CO-C₁₋₂₀ alkyl, CO-C₂₋₂₀ alkenyl, CO-C₅₋₁₀ aryl and CO-C₅₋₁₀ ar-C₁₋₂o alkyl. For example, the compounds of SEQ ID NO:9 (code 6433), SEQ ID NO:32 (code 6464), SEQ ID NO:51 (code 6646), SEQ ID NO:54 (code 6651), SEQ ID NO:55 (code 6652) and SEQ ID NO:56 (code 6653) have a longer half-life in the blood than native human PP. The compounds of SEQ ID NO:52 (code 6647), SEQ ID NO:53 (code 6650), SEQ ID NO:56 (code 6653) and SEQ ID NO:57 (code 6654) are stronger binders to the Y4 receptor than native human PP. Preferred acyl groups are CO-C₁₋₂₀ alkyl and CO-C₂₋₂₀ alkenyl, for example CO-C₁₋₂₀ alkyl, in particular lauryl or stearoyl, especially lauryl.

Preferred additional amino acids at position 0 are Ala and Pro. In a further embodiment, the Ala at position 1 is furnished with an additional amino acid at position 0 selected from Pro, Gly, His, Ala and Tyr; especially Pro and Gly.

In a preferred embodiment, analogues have the Ala at position 1 furnished with an additional amino acid at position 0 as described above. Such analogues have a total length of 37 amino acids (unless there are further extensions or deletions). It is found that they have a longer half-life in the blood than native human PP. There is evidence to suggest that analogues extended at the 0 position are less susceptible to cleavage by DPP IV than peptides without the extension. Furthermore, it is postulated that cleavage of a 37 amino acid long analogue at the N-terminus by DPP IV leads to generation of a 35 amino acid analogue which retains activity at the receptor (such a compound is a compound of the current invention in which a) the Ala at position 1 is (iii) absent). It has been found that a 34 amino acid long PP analogue (the product of the cleavage away of the two N-terminal amino acids from a 36 amino acid PP) retains less activity than the 35 amino acid compound.

In an embodiment, analogues which a) have the Ala at position 1 altered as in a) above, the Ala at position 1 is absent. The compound of Example 11 (code 643 5) has a longer half-life in the blood than native human PP.

In a further embodiment, the analogue has the Leu at position 3 replaced by the alternative amino acid Ile or Ser. The analogues have a longer half-life in the blood than native human PP and/or they have stronger binding to the Y4 receptor than native human PP. For Example, the compounds of SEQ ID NO:42 (code 6618) and SEQ ID NO:43 (code 6619) have a longer half-life in the blood than native human PP. For Example, the compounds of SEQ ID NO:586 (code 4057640) and SEQ ID NO:587 (code 4057641) have stronger binding to the Y4 receptor than native human PP.

In a further embodiment, the analogue has the Glu at position 4 replaced by the alternative amino acid Lys. The analogues have a longer half-life in the blood than native human PP and/or they have stronger binding to the Y4 receptor than native human PP. For Example, the compounds of SEQ ID NO: 19 (code 6446) has a longer half-life in the blood than native human PP. For Example, the compounds of SEQ ID NO:586 (code 4057640) and SEQ ID NO:587 (code 4057641) have stronger binding to the Y4 receptor than native human PP.

In a further embodiment, the analogue has the Pro at position 5 replaced by the alternative amino acid Ala. The analogues have a longer half-life in the blood than native human PP and/or they have stronger binding to the Y4 receptor than native human PP. For Example, the compound of SEQ ID NO:702 (code 4058286) has stronger binding to the Y4 receptor than native human PP.

In a further embodiment, the analogue has the Val at position 6 replaced by an alternative amino acid selected from Glu, His, Ile, Leu, Ser, Cys, Phe, Lys, Thr, Ala, Arg, Asp, Lys, Tyr and Met. The analogues have a longer half-life in the blood than native human PP and/or they have stronger binding to the Y4 receptor than native human PP. For Example, the compounds of SEQ ID NO:275 (code 4055745) and SEQ ID NO:276 (4055746) have a longer half-life in the blood than native human PP and they have stronger binding to the Y4 receptor than native human PP. For Example, the compounds of SEQ ID NO:256 (code 4055710)_{;} SEQ ID NO:257 (code 4055711) and SEQ ID NO:258 (code 4055712) have stronger binding to the Y4 receptor than the compound of SEQ ID NO: 18 (code 6445).

Preferred amino acids for replacement of Val at position 6 are Phe and Glu, especially Phe.

In a further embodiment, the analogue has the Tyr at position 7 replaced by the alternative amino acid Ala, Asn or Phe. The analogues have a longer half-life in the blood than native human PP and/or they have stronger binding to the Y4 receptor than native human PP. For Example, the compounds of SEQ ID NO:1092 (code 4059668) has a longer half-life in the blood than native human PP and it has stronger binding to the Y4 receptor than native human PP. For Example, the compounds of SEQ ID NO:929 (code 4059104), SEQ ID NO: 1090 (code 4059937) and SEQ ID NO: 1092 (code 4059939) have stronger binding to the Y4 receptor than native human PP.

In a further embodiment, the analogue has the Asn at position 11 replaced by an alternative amino acid selected from Asp and Tyr. For Example, the compound of SEQ ID NO:104 (code 6841) has have a longer half-life in the blood than the compound of SEQ ID NO:102 (code 6839).

In a further embodiment, the analogue has one or more of the native amino acids 14 to 26 (first row, bold) replaced with an amino acid selected from the amino acids in the respective column below it:

| | | | | | | | | | | | | |
|---|---|---|---|---|---|---|---|---|---|---|---|---|
| 14 | 15 | 16 | 17 | 18 | 19 | 20 | 21 | 22 | 23 | 24 | 25 | 26 |
| **Pro** | **Glu** | **Gln** | **Met** | **Ala** | **Gln** | **Tyr** | **Ala** | **Ala** | **Asp** | **Leu** | **Arg** | **Arg** |
| Glu | Gln | Glu | Leu | Asn | Arg | | Tyr | Ser | Ala | Met | Lys | His |
| | | Asp | NLeu | Val | Lys | | Phe | Ile | Glu | Cys | NLeu | Lys |
| | | Lys-Acyl* | Ile | Ser | His | | Glu | Val | Ser | NLeu | | |
| | | | Arg | | Glu | | | Thr | Gly | | | |
| | | | Thr | | | | | | Gln | | | |
| | | | Gln | | | | | | Ile | | | |
| | | | Glu | | | | | | Leu | | | |
| | | | Lys | | | | | | NLeu | | | |
| | | | Val | | | | | | Val | | | |

| | | | | | | | | | | | | |
|---|---|---|---|---|---|---|---|---|---|---|---|---|
| * the acyl group being selected from: CO-C₁₋₂₀ alkyl, CO-C₂-₂₀ alkenyl, CO-C₅₋₁₀ aryl and CO-C₅₋₁₀ ar-C₁₋₂₀ alkyl | | | | | | | | | | | | |

The analogues have a longer half-life in the blood than native human PP and/or they have stronger binding to the Y4 receptor than native human PP. Particularly preferred are the the analogue has one or more of the native amino acids 16 to 23 (first row, bold) replaced with an amino acid selected from the amino acids in the respective column below it:

| | | | | | | | |
|---|---|---|---|---|---|---|---|
| 16 | 17 | 18 | 19 | 20 | 21 | 22 | 23 |
| **Gln** | **Met** | **Ala** | **Gln** | **Tyr** | **Ala** | **Ala** | **Asp** |
| Glu | Leu | Asn | Arg | | Tyr | Ser | Ala |
| Asp | Arg | Val | Lys | | | | Glu |
| | | | His | | | | Ser |

Those analogues have particularly strong binding to the Y4 receptor.

Particularly preferred motifs in the 16 to 23 region include:
Asp Met Ala Arg Tyr Tyr Ser Ala
Glu Leu Asn Arg Tyr Tyr Ser Ala
Glu Leu Val Lys Tyr Tyr Ser Ala
Glu Leu Val Lys Tyr Tyr Ala Ser
Glu Leu Asn Arg Tyr Tyr Ala Ser
GIn Met Ala Arg Tyr Tyr Ser Ala
Gln Met Ala Arg Tyr Ala Ala Ala
Gln Met Ala Arg Tyr Ala Ala Glu
Gln Met Ala Arg Tyr Ala Ala Asp
Gln Met Ala Arg Tyr Tyr Ser Glu
Gln Met Ala Gln Tyr Ala Ala Ala
Gln Met Ala Gln Tyr Ala Ala Glu
Gln Met Ala His Tyr Ala Ala Ala
Gln Met Ala His Tyr Ala Ala Glu
Gln Met Ala Lys Tyr Ala Ala Asp

In particular:
Asp Met Ala Arg Tyr Tyr Ser Ala
Glu Leu Asn Arg Tyr Tyr Ser Ala
Glu Leu Val Lys Tyr Tyr Ser Ala
Glu Leu Val Lys Tyr Tyr Ala Ser
Glu Leu Asn Arg Tyr Tyr Ala Ser
Gln Met Ala Arg Tyr Tyr Ser Ala
Gln Met Ala Arg Tyr Ala Ala Ala
Gln Met Ala Arg Tyr Ala Ala Glu
Gln Met Ala Arg Tyr Tyr Ser Glu
Gln Met Ala Gln Tyr Ala Ala Glu
Gln Met Ala His Tyr Ala Ala Ala
Gln Met Ala His Tyr Ala Ala Glu
Gln Met Ala Lys Tyr Ala Ala Asp

A further particularly preferred set of analogue has one or more of the native amino acids 15 to 25 (first row, bold) replaced with an amino acid selected from the amino acids in the respective column below it:

| 15 | 16 | 17 | 18 | 19 | 20 | 21 | 22 | 23 | 24 | 25 |
|---|---|---|---|---|---|---|---|---|---|---|
| **Glu** | **Gln** | **Met** | **Ala** | **Gln** | **Tyr** | **Ala** | **Ala** | **Asp** | **Leu** | **Arg** |
| Gln | Glu | Leu | Asn | Arg | | Tyr | Ser | Ala | Met | Lys |
| | | | Val | Lys | | | | Glu | | |
| | | | | His | | | | Ser | | |

The analogues have a particularly long half-life in the blood.

Particularly preferred motifs in the 15 to 25 region include:
Glu Gln Met Ala Gln Tyr Ala Ala Asp Leu Arg
Glu Gln Met Ala Gln Tyr Ala Ala Ala Leu Arg
Glu Gln Met Ala Lys Tyr Ala Ala Asp Leu Lys
Glu Gln Met Ala Arg Tyr Tyr Ser Glu Leu Arg
Glu Gln Met Ala Arg Tyr Tyr Ser Ala Leu Arg
Glu Gln Met Ala Arg Tyr Ala Ala Asp Leu Lys
Glu Gln Leu Ala Gln Tyr Ala Ala Asp Leu Arg
Glu Glu Leu Asn His Tyr Tyr Ala Ser Leu Arg
Glu Glu Leu Val Lys Tyr Tyr Ala Ser Leu Arg
Gln Gln Met Ala Gln Tyr Ala Ala Glu Leu Arg
Gln Gln Met Ala Gln Tyr Ala Ala Glu Met Arg

In particular:
Glu Gln Met Ala Gln Tyr Ala Ala Asp Leu Arg
Glu Gln Met Ala Lys Tyr Ala Ala Asp Leu Lys
Glu Gln Met Ala Arg Tyr Tyr Ser Glu Leu Arg
Glu Gln Met Ala Arg Tyr Tyr Ser Ala Leu Arg
Glu Gln Met Ala Arg Tyr Ala Ala Asp Leu Lys
Glu Gln Leu Ala Gln Tyr Ala Ala Asp Leu Arg
Glu Glu Leu Asn His Tyr Tyr Ala Ser Leu Arg
Glu Glu Leu Val Lys Tyr Tyr Ala Ser Leu Arg
Gln Gln Met Ala Gln Tyr Ala Ala Glu Leu Arg
Gln Gln Met Ala Gln Tyr Ala Ala Glu Met Arg

A further particularly preferred set of analogue has one or more of the native amino acids 15 to 26 (first row, bold) replaced with an amino acid selected from the amino acids in the respective column below it:

| 1 5 | 16 | 17 | 18 | 19 | 20 | 21 | 22 | 23 | 24 | 25 | 26 |
|---|---|---|---|---|---|---|---|---|---|---|---|
| **Glu** | **Gln** | **Met** | **Ala** | **Gln** | **Tyr** | **Ala** | **Ala** | **Asp** | **Leu** | **Arg** | **Arg** |
| Gln | Glu | Leu | Val | Arg | | Tyr | Ser | Ala | | Lys | Lys |
| | | Ile | | Lys | | | | Glu | | | |
| | | | | Glu | | | | Ser | | | |

The analogues have a particularly long half-life in the blood.

Particularly preferred motifs in the 15 to 26 region include:
Glu Gln Met Ala Gln Tyr Ala Ala Ala Leu Arg Arg
Glu Gln Met Ala Arg Tyr Tyr Ser Ala Leu Arg Arg
Glu Gln Met Ala Glu Tyr Ala Ala Asp Leu Arg Arg
Glu Gln Met Ala Lys Tyr Ala Ala Asp Leu Lys Arg
Glu Gln Leu Ala Gln Tyr Ala Ala Asp Leu Arg Arg
Glu Gln Leu Ala Gln Tyr Ala Ala Asp Leu Arg Lys
Glu Glu Ile Val Lys Tyr Tyr Ser Ala Leu Arg Arg
Glu Glu Leu Val Lys Tyr Tyr Ala Ser Leu Arg Arg
Gln Gln Met Ala Gln Tyr Ala Ala Glu Leu Arg Lys
Gln Gln Met Ala Gln Tyr Ala Ala Glu Leu Arg Arg
Gln Gln Leu Ala Gln Tyr Ala Ala Glu Leu Arg Arg

In particular:
Glu Gln Met Ala Arg Tyr Tyr Ser Ala Leu Arg Arg
Glu Gln Met Ala Glu Tyr Ala Ala Asp Leu Arg Arg
Glu Gln Met Ala Lys Tyr Ala Ala Asp Leu Lys Arg
Glu Gln Leu Ala Gln Tyr Ala Ala Asp Leu Arg Arg
Glu Gln Leu Ala Gln Tyr Ala Ala Asp Leu Arg Lys
Glu Glu Ile Val Lys Tyr Tyr Ser Ala Leu Arg Arg
Glu Glu Leu Val Lys Tyr Tyr Ala Ser Leu Arg Arg
Gln Gln Met Ala Gln Tyr Ala Ala Glu Leu Arg Lys
Gln Gln Met Ala Gln Tyr Ala Ala Glu Leu Arg Arg
Gln Gln Leu Ala Gln Tyr Ala Ala Glu Leu Arg Arg

In an embodiment, the analogue has at positions 16 to 23 the sequence:
G1n Met Ala Lys Tyr Ala Ala Ala

Preferred analogues further include those with:
- Ala at position 23
- Arg Tyr Tyr Ser Glu at positions 19 to 23
- Lys Tyr Ala Ala Ala at positions 19 to 23
- Leu at position 17
- Nleu at position 17
- Glu at position 23
- Arg at position 19
- Lys at position 19
- Lys at position 19 and Ala at position 23
- Arg at position 19 and Ala at position 23
- Arg at position 19 and Glu at position 23
- His at position 19 and Glu at position 23

In a further embodiment, the analogue has at positions 29, 30 and 31:
(i) the Met at position 30 replaced by an alternative amino acid selected from Leu, Arg, Glu, His, NLeu, Ile, Val, Phe, Thr, Asn, Cys and Lys; and/or
(ii) the Leu at position 31 replaced by an alternative amino acid selected from Ile and Val; and/or
(iii) the Asn at position 29 replaced by Asp.

The analogues have a longer half-life in the blood than native human PP and/or they have stronger binding to the Y4 receptor than native human PP. For Example, the compounds of SEQ ID NO:259 (code 4055713), SEQ ID NO:262 (code 4055716) and SEQ ID NO:283 (code 4055857) have a longer half life in the blood than native human PP. For Examples, the compounds of SEQ ID NO:259 (code 4055713), SEQ ID NO:260 (code 4055714) and SEQ ID NO:261 (code 4055715) have stronger binding to the Y4 receptor than native human PP.

Preferred analogues have the Met at position 30 replaced by Lys, N-Leu, Val, Ile or Leu, for example Lys, N-Leu, Ile or Leu, especially Lys. Further preferred analogues have the Met at position 30 replaced by Lys, N-Leu, Ile or Leu, especially Lys, and have Leu at position 31.

Further preferred analogues have the Asn at position 29 replaced by Asp.

In a further embodiment, the analogue has at positions 34, 35 and 36:
(i) the Pro at position 34 replaced by the alternative amino acid Gln, Asn, DPro, Leu, His or NVa (Nor-valine); and/or
(i) the Arg at position 35 replaced by the alternative amino acid G1n or HArg; and/or
(ii) the Tyr at position 36 replaced by the alternative amino acid Phe

The analogues have a longer half-life in the blood than native human PP and/or they have stronger binding to the Y4 receptor than native human PP. For Example, the compounds of SEQ ID NO:24 (code 6453), SEQ ID NO:62 (code 6659), SEQ ID NO:255 (code 4055708) and SEQ ID NO:283 (code 4055857) have stronger binding to the Y4 receptor than native human PP, and the compounds of SEQ ID NO:255 (code 4055708) and SEQ ID NO:283 (code 4055857) have a longer half life in the blood than native human PP.

Preferred analogues have Gln or NVa at position 34 and/or Phe at position 36.

In a further embodiment, the analogue is dimerised by disulphide bond formation between a pair of Cys residues added to the C termini of the two units, or as replacements for Leu at position 31 of the two units.

For Example, the compounds of SEQ ID NO: 100 (code 6835) and SEQ ID NO:285 (code 4055860) have a longer half life in the blood than native human PP.

In a preferred embodiment, the analogue of the invention comprises a combination of the changes listed in a) to g) above. Particularly preferred analogues have:
- a change in the 14 to 26 region (d above) and a change in the 30 and 31 region (e above)
- a change at position 6 (b above), a change at position 11 (c above), a change in the 14 to 26 region (d above) and a change in the 30 and 31 region (e above)
- a change at position 1 (a above) and a change in the 30 and 31 region (e above)
- a change at position 1 (a above), a change in the 14 to 26 region (d above) and a change in the 30 and 31 region (e above)

For example, an analogue of the invention may have an additional Ala at position 0, and a Lys at position 30. The compound of SEQ ID NO: 171 (code 7218) binds the Y4 receptor better than either of the compounds of SEQ ID NO:9 (Ala 0, code 6433) or SEQ ID NO:281 (Lys 30, code 4055855). For Example, an analogue of the invention may have an additional Ala at position 0, a Lys at position 19 and a Lys at position 30. The compound of SEQ ID NO:348 (Ala 0, Lys19, Lys 30, code 4056385) binds the Y4 receptor better and has a longer half life in the blood than any of the compounds of SEQ ID NO: 171 (Ala 0, Lys 19, code 7218), SEQ ID NO:9 (Ala 0, code 6433), SEQ ID NO:224 (Lys 19, code 4055291) or SEQ ID NO:281 (Lys 30, code 4055855).

For example, an analogue of the invention may have an Ala at position 23 and a Lys at position 30. The compound of SEQ ID NO: 167 (code 7214) binds the Y4 receptor better and has a longer half life in the blood than either of the compounds of SEQ ID NO:277 (Ala 23, code 4055747) or SEQ ID NO:281 (Lys 30, code 4055855). For Example, an analogue may further have an additional Ala at position 0. The compound of SEQ ID NO:168 (Ala 0, Ala 23 and Lys 30, code 7215) has still further better binding at the Y4 receptor.

For example, an analogue of the invention may have an additional Ala at position 0, and a Leu at position 30. The compound of SEQ ID NO:59 (code 6656) has a longer half life in the blood than either of the compounds of SEQ ID NO:9 (Ala 0, code 6433) or the compound with Leu at position 30 and no other changes from native PP (data not shown). In a further preferred embodiment, the analogue of the invention comprises a combination of the changes listed in a) to g) above in which the native Gln at position 19 is replaced, especially by Lys, His or Arg (d above); the native Met at position 30 replaced, especially by Lys, His or Arg (e above); and the native Asp at position 23 is replaced, especially by Glu or Ala (d above).

It has in particular been found that analogues of PP in which the Met at position 30 is replaced by Lys, His or Arg are especially beneficial in having a longer half-life in the blood than native human PP and/or stronger binding to the Y4 receptor than native human PP.

It has further particularly been found that analogues of PP in which the Gln at position 19 is replaced by Lys, His or Arg are especially beneficial in having a longer half-life in the blood than native human PP and/or stronger binding to the Y4 receptor than native human PP.

Accordingly, a preferred set of analogues has the Gln at position 19 and the Met at position 30 replaced by Lys, His or Arg. A preferred set of compounds is thus defined by the amino acid sequence:
Xaa0-Xaal -Pro-Leu-Glu-Pro-Xaa6-Xaa7-Pro-Gly-Xaa10-Xaa11-Ala-Xaa13-Pro-Xaa15-Xaa16-Xaa17-Xaa18-Xaa19-Tyr-xaa21-Xaa22-Xaa23-Leu-Arg-Arg-Tyr-Ile-Xaa29-Xaa30-Leu-Thr-Arg-Xaa34-Arg-Xaa36-NH2,

Wherein:
Xaa0-Xaal is Pro-Ala, Gly-Ala, His-Ala, Ala-Ala, Tyr-Ala, Ala or absent;
Xaa6 is Glu, Ser, Thr, Val, His, Lys, His, Ile or Asp;
Xaa7 is Tyr or Ala;
Xaal0 is Asp or Glu;
Xaa1 1 is Asn or Asp;
Xaa13 is Thr or Ser;
Xaa15 is Glu or Gln;
Xaa16 is Glu, Gln or Asp;
Xaa17 is Met, Leu, Lys or Ile;
Xaa18 is Ala or Asn;
Xaa19 is Arg, Lys or His;
Xaa21 is Ala or Tyr;
Xaa22 is Ala or Ser;
Xaa23 is Glu, Ala or Asp;
Xaa29 is Asp or Asn;
Xaa30 is Lys, His or Arg;
Xaa34 is N-Val or Pro; and
Xaa is Tyr or Phe.

In one embodiment, Xaa0-Xaa1 is Pro-Ala, Gly-Ala or absent.

In one embodiment, Xaa7 is Tyr.

In one embodiment, Xaa10 is Asp.

In one embodiment, Xaa1 is Asn. In an alternative embodiment, Xaa11 is Asp.

In one embodiment, Xaa13 is Thr.

In one embodiment, Xaa15 is Glu. In an alternative embodiment Xaa15 is Gln.

In one embodiment, Xaa16 is Glu or Gln. For example Xaa16 may be Glu or Xaa16 may be Gln.

In one embodiment, Xaa17 is Met, Leu or Lys. For example.Xaa17 may be Met, Xaa17 may be Leu or Xaa17 may be Lys.

In one embodiment, Xaal8 is Ala.

In one embodiment, Xaa19 is Arg. In alternative embodiment, Xaa19 is Lys. In an alternative embodiment, Xaa19 is His.

In one embodiment, Xaa 21 is Ala. In alternative embodiment, Xaa21 is Tyr.

In one embodiment, Xaa22 is Ala. In alternative embodiment, Xaa22 is Ser.

In one embodiment, Xaa23 is Glu or Ala. For Example, Xaa23 may be Glu, or Xaa23 may be Asp. A particularly preferred set of analogues has the native Gln at position 19, the native Met at position 30 replaced by Lys, His or Arg, and the native Asp at position 23 replaced by Glu or Ala.

In one embodiment, Xaa29 is Asp. In an alternative embodiment Xaa29 is Asn.

In one embodiment, Xaa30 is Lys or His. For Example Xaa 30 may be Lys or Xaa30 may be Arg.

In one embodiment, Xaa 34 is Pro.

In one embodiment, Xaa36 is Tyr. In an alternative embodiment Xaa is Phe

Particularly preferred compounds of the invention that fulfill these criteria are the compounds with code numbers:
4056796, 4056797, 4056801, 4057018, 4057111, 4057113, 4057118, 4057134, 4057136, 4057137, 4057138, 4057151, 4057231, 4057258, 4057269, 4057440, 4057598, 4057603, 4057604, 4057611 4057612, 4057630, 4057634, 4057637, 4057643, 4057753, 4057766, 4058072, 4058079, 4058149, 4058166, 4058167, 4058281, 4058369, 4058370, 4058371, 4058376, 4058377, 4058379, 4058492, 4058503, 4058505, 4058506, 4058582, 4058667, 4058668, 4058671, 4058676, 4058691, 4058705, 4058911, 4058912, 4059008, 4059013, 4059025, 4059350, 4059351, 4059361, 4059362, 4059363, 4059370, 4059376, 4059384, 4059385, 4059387, 4059468, 4059469, 4059653, 4059665, 4059666, 4060204, 4060272, 4060274, 4060282, 4060284, 4060316, 4060358, 4060680, 4060684, 4060685, 4060686, 4060689, 4060893, 4061090 and 4061236.

The invention further provides a compound that is an analogue (II) of human Pancreatic Polypeptide (SEQ. ID No 1) which differs from native human pancreatic polypeptide in one of more of the following respects:

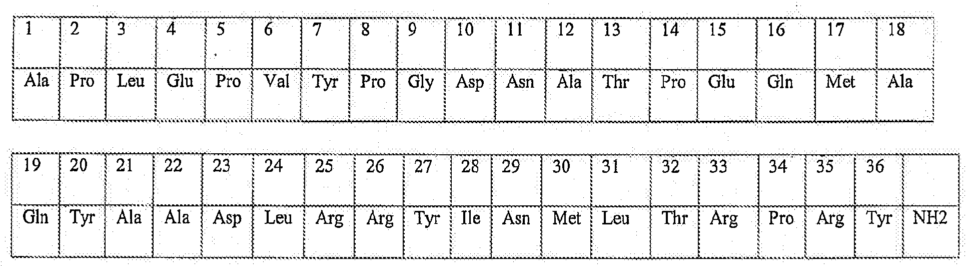

a) it has the Ala at position 1
(i) replaced by an alternative amino acid selected from D-Ala, Asp, Glu, Gly, His, Tyr, Lys, acylated Ala and acylated Lys, the acyl group being selected from: CO― C₁₋₂₀ alkyl, CO―C₂₋₂₀ alkenyl, CO-C₅₋₁₀ aryl and CO―C₅₋₁₀ ar―C₁₋₂₀ alkyl, and/or
(ii) furnished with an additional amino acid at position 0, selected from Gly, Lys, His, Glu, Asp, Pro, Ile, Phe, Val,Cys, Tyr, TyrTyrTyr, Ala, D-Ala, and acylated Ala, the acyl group being selected from: CO-C₁₋₂₀ alkyl, CO-C₂₋₂₀ alkenyl, CO―C₅₋₁₀ aryl and CO―C₅₋₁₀ ar―C₁₋₂₀ alkyl; or
(iii) absent
b) at positions 3, 4, 6, 7 and 10, it has
(i) the Leu at position 3 replaced by the alternative amino acid Ile or Ser; and/or
(ii) the Glu at position 4 replaced by the alternative amino acid Lys; and/or
(iii) the Val at position 6 replaced by an alternative amino acid selected from Glu, His, Ile, Leu, Ser, Phe, Cys, Thr and Met; and/or
(iv) the Tyr at position 7 replaced by the alternative amino acid Ala or Phe; and/or
(v) the Asp at position 10 replaced by the alternative amino acid Glu;
c) it has the Asn at position 11 replaced by an alternative amino acid selected from Asp and Tyr; and/or the Thr at position 13 replaced by the alternative amino acid Ser;
d) at positions 14 to 26, it has one or more of the native amino acids 14 to 26 (first row, bold) replaced with an amino acid selected from the amino acids in the respective column below it:

| 14 | 15 | 16 | 17 | 18 | 19 | 20 | 21 | 22 | 23 | 24 | 25 | 26 |
|---|---|---|---|---|---|---|---|---|---|---|---|---|
| **Pro** | **Glu** | **Gln** | **Met** | **Ala** | **Gln** | **Tyr** | **Ala** | **Ala** | **Asp** | **Leu** | **Arg** | **Arg** |
| Glu | Gln | Glu | Leu | Asn | Arg | | Tyr | Ser | Ala | Met | Lys | His |
| | | Asp | NLeu | Val | Lys | | Phe | Ile | Glu | Cys | NLeu | Lys |
| | | Lys-Acyl* | Ile | | His | | Glu | Val | Ser | | | |
| | | | Arg | | Glu | | | Thr | Gly | | | |
| | | | Thr | | | | | | Gln | | | |
| | | | Gln | | | | | | Ile | | | |
| | | | Glu | | | | | | Leu | | | |
| | | | Lys | | | | | | NLeu | | | |
| | | | | | | | | | Val | | | |

| | | | | | | | | | | | | |
|---|---|---|---|---|---|---|---|---|---|---|---|---|
| * the acyl group being selected from: CO-C₁₋₂₀ alkyl, CO―C₂₋₂₀ alkenyl, CO―C₅₋₁₀ aryl and CO―C₅₋₁₀ ar―C₁₋₂₀ alkyl | | | | | | | | | | | | |

e) at positions 30 and 31, it has:
(i) the Met at position 30 replaced by an alternative amino acid selected from Leu, Arg, Glu, His, NLeu, Ile, Val, Phe, Thr, Asn, Cys and Lys; and/or
(ii) the Leu at position 31 replaced by an alternative amino acid selected from Ile and Val
f) at positions 34, 35 and 36, it has:
(i) the Pro at position 34 replaced by the alternative amino acid Gln, Asn, DPro, Leu, His or NVa (Nor-valine); and/or
(ii) the Arg at position 35 replaced by the alternative amino acid Gln or HArg; and/or
(iii) the Tyr at position 36 replaced by the alternative amino acid Phe
g) it is dimerised by disulphide bond formation between a pair of Cys residues added to the two units at the C termini or as replacements for Leu at position 31 in the two units,
h) it is cyclised by disulphide band formation between a pair of Cys residues added as a replacement amino acid residue at a pair of positions selected from the following list: 8 & 17, 8 & 20, 5 & 24, 2 & 27, or between a Cys residue added at a replacement amino acid at position 28 or 29 and a Cys residue added as an additional amino acid at position 0; whereby when the analogue has Ala1 absent then it has at least one further change other than one or more selected from Nle17 and N1e30,
whereby when the analogue has one or more,of N1e17, N1e30 and His34, then it has at least one further change other than the one or more selected from N1e17, Nle30 and His34,
and
whereby when the analogue has Gln at position 34, then it has at least one further change other than one or more selected from Ile31, Val31, Leu30 and NLeu30,
or a variant or derivative thereof; or a salt or solvate thereof,
and whereby if the analogue has one or more of:
- Arg at position 19 (optionally with Phe at position 6)
- Tyr or Glu at position 21 (optionally with Phe at position 6)
- Ser at position 22 (optionally with Phe at position 6)
- Ala or Gln at position 23 (optionally with Phe at position 6)
or if the analogue has one of:
- Glu at position 23 and Glu at position 6
- Asp at position 11 and Gln at position 15 and Glu at position 23 and Met at position 24
- Met at position 6 and Tyr at position 11 and Glu at position 21 and Thr at position 22 and
- Gln at position 23 and Thr at position 30
then the analogue has at least one further difference from the native human Pancreatic Polypeptide.

The invention provides a compound of formula (II) for use as a medicament.

Preferred compounds of the invention are those described above.

### Further specific embodiments

According to a particular preferred embodiments the invention the analogue of human Pancreatic Polypeptide (SEQ. ID No 1) differs from native human pancreatic polypeptide in one of more of the following respects:

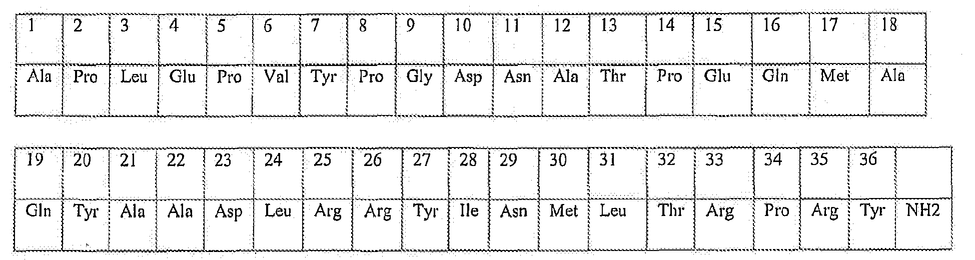

a) it has the Ala at position 1
(i) replaced by an alternative amino acid selected from D-Ala, Asp, Glu, Gly, His, Tyr, Lys, acylated Ala and acylated Lys, the acyl group being selected from: CO― C₁₋₂₀ alkyl, CO―C₂₋₂₀ alkenyl, CO―C₅₋₁₀ aryl and CO―C₅₋₁₀ ar―C₁₋₂₀ alkyl, and/or
(ii) furnished with an additional amino acid at position 0, selected from Gly, Lys, His, Glu, Asp, Pro, Ile, Phe, Val, Cys, Tyr, TyrTyrTyr, Ala, D-Ala, and acylated Ala, the acyl group being selected from: CO―C₁₋₂₀ alkyl, CO―C₂₋₂₀ alkenyl, CO―C₅₋₁₀ aryl and CO―C₅₋₁₀ ar―C₁₋₂₀ alkyl; or
(iii) absent
b) at positions 3, 4, 6, 7 and 10, it has
(i) the Leu at position 3 replaced by the alternative amino acid Ile or Ser; and/or
(ii) the Glu at position 4 replaced by the alternative amino acid Lys; and/or
(iii) the Val at position 6 replaced by an alternative amino acid selected from Glu, His, Ile, Leu, Ser, Phe, Cys, Thr and Met; and/or
(iv) the Tyr at position 7 replaced by the alternative amino acid Ala or Phe; and/or
(v) the Asp at position 10 replaced by the alternative amino acid Glu;
c) it has the Asn at position 11 replaced by an alternative amino acid selected from Asp and Tyr; and/or the Thr at position 13 replaced by the alternative amino acid Ser;
d) at positions 14 to 26, it has one or more of the native amino acids 14 to 26 (first row, bold) replaced with an amino acid selected from the amino acids in the respective column below it:

| 14 | 15 | 16 | 17 | 18 | 19 | 20 | 21 | 22 | 23 | 24 | 25 | 26 |
|---|---|---|---|---|---|---|---|---|---|---|---|---|
| **Pro** | **Glu** | **Gln** | **Met** | **Ala** | **Gln** | **Tyr** | **Ala** | **Ala** | **Asp** | **Leu** | **Art** | **Arg** |
| Glu | Gln | Glu | Leu | Asn | Arg | | Tyr | Ser | Ala | Met | Lys | His |
| | | Asp | NLeu | Val | Lys | | Phe | Ile | Glu | Cys | NLeu | Lys |
| | | Lys-Acyl * | Ile | | His | | Glu | Val | Ser | | | |
| | | | Arg | | Glu | | | Thr | Gly | | | |
| | | | Thr | | | | | | Gln | | | |
| | | | Gln | | | | | | Ile | | | |
| | | | Glu | | | | | | Leu | | | |
| | | | Lys | | | | | | NLeu | | | |
| | | | | | | | | | Val | | | |

| | | | | | | | | | | | | |
|---|---|---|---|---|---|---|---|---|---|---|---|---|
| * the acyl group being selected from: CO―C₁₋₂₀ alkyl, CO―C₂₋₂₀ alkenyl, CO―C₅₋₁₀ aryl and CO―C₅₋₁₀ ar―C₁₋₂₀ alkyl | | | | | | | | | | | | |

e) at positions 29, 30 and 31, it has:
(i) the Met at position 30 replaced by an alternative amino acid selected from Leu, Arg, Glu, His, NLeu, Ile, Val, Phe, Thr, Asn, Cys and Lys; and/or
(ii) the Leu at position 31 replaced by an alternative amino acid selected from Ile and Val; and/or
(iii) the Asn at position 29 replaced by Asp
f) at positions 34, 35 and 36, it has:
(i) the Pro at position 34 replaced by the alternative amino acid Gln, Asn, DPro, Leu, His or NVa (Nor-valine); and/or
(ii) the Arg at position 35 replaced by the alternative amino acid Gln or HArg; and/or
(iii) the Tyr at position 36 replaced by the alternative amino acid Phe
g) it is dimerised by disulphide bond formation between a pair of Cys residues added to the two units at the N termini or as replacements for Leu at position 31 in the two units,
h) it is cyclised by disulphide bond formation between a pair of Cys residues added as a replacement amino acid residue at a pair of positions selected from the following list: 8 & 17, 8 & 20, 5 & 24, 2 & 27, or between a Cys residue added at a replacement amino acid at position 28 or 29 and a Cys residue added as an additional amino acid at position 0; whereby when the analogue has Ala1 absent then it has at least one further change other than one or more selected from N1e17 and N1e30,
whereby when the analogue has one or more of N1e17, N1e30 and His34, then it has at least one further change other than the one or more selected from N1e17, N1e30 and His34, and
whereby when the analogue has Gln at position 34, then it has at least one further change other than one or more selected from Ile31, Val31, Leu30 and NLeu30.

Optionally, if the analogue has one or more of:
- Arg at position 19 (optionally with Phe at position 6)
- Tyr or Glu at position 21 (optionally with Phe at position 6)
- Ser at position 22 (optionally with Phe at position 6)
- Ala or Gln at position 23 (optionally with Phe at position 6)
or if the analogue has one of:
- Glu at position 23 and Glu at position 6
- Asp at position 11 and Gln at position 15 and Glu at position 23 and Met at position 24
- Met at position 6 and Tyr at position 11 and Glu at position 21 and Thr at position 22 and
- Gln at position 23 and Thr at position 30
then the analogue has at least one further difference from the native human Pancreatic Polypeptide.

The analogues may optionally be for use (i) in the treatment (including prophylactic treatment) of obesity or diabetes in a subject, or (ii) in the reduction of appetite in a subject, the reduction of food intake in a subject, or in the reduction of calorie intake in a subject.

Further particular embodiments of the invention are directed to an analogue of human Pancreatic Polypeptide (SEQ. ID No 1) which differs from native human pancreatic polypeptide in one of more of the following respects:

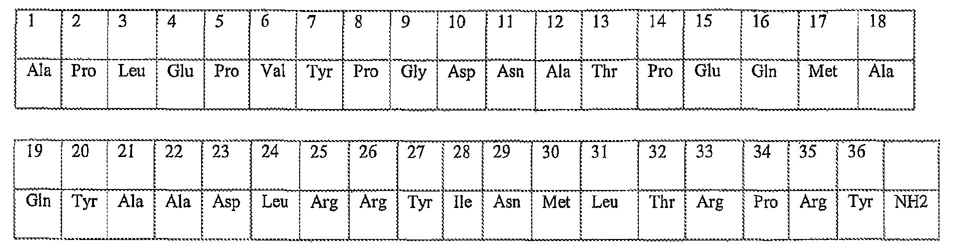

a) it has the Ala at position 1
(i) replaced by an alternative amino acid selected from D-Ala, Lys, acylated Ala and acylated Lys, the acyl group being selected from: CO―C₁₋₂₀ alkyl, CO―C₂₋₂₀ alkenyl, CO―C₅₋₁₀ aryl and CO―C₅₋₁₀ ar―C₁₋₂₀ alkyl, and/or
(ii) furnished with an additional amino acid at position 0, selected from Gly, Lys, His, Glu, Asp, Pro, Tyr, TyrTyrTyr, Ala, D-Ala, and acylated Ala, the acyl group being selected from: CO―C₁₋₂₀ alkyl, CO―C₂₋₂₀ alkenyl, CO―C₅₋₁₀ aryl and CO―C₅₋₁₀ ar―C₁₋₂₀ alkyl; or
(iii) absent
b) it has the Val at position 6 replaced by an alternative amino acid selected from Glu, Phe, Lys, Thr and Met;
c) it has the Asn at position 11 replaced by an alternative amino acid selected from Asp and Tyr;
d) at positions 14 to 26, it has one or more of the native amino acids 14 to 26 (first row, bold) replaced with an amino acid selected from the amino acids in the respective column below it:

| 14 | 15 | 16 | 17 | 18 | 19 | 20 | 21 | 22 | 23 | 24 | 25 | 26 |
|---|---|---|---|---|---|---|---|---|---|---|---|---|
| **Pro** | **Glu** | **Gln** | **Met** | **Ala** | **Gln** | **Tyr** | **Ala** | **Ala** | **Asp** | **Leu** | **Arg** | **Arg** |
| Glu | Gln | Glu | Leu | Asn | Arg | | Tyr | Ser | Ala | Met | Lys | His |
| | | Asp | NLeu | Val | Lys | | Phe | Ile | Glu | | NLeu | Lys |
| | | Lys-Acyl* | Ile | | His | | Glu | Val | Ser | | | |
| | | | Arg | | Glu | | | Thr | Gly | | | |
| | | | Thr | | | | | | Gln | | | |

| | | | | | | | | | | | | |
|---|---|---|---|---|---|---|---|---|---|---|---|---|
| * the acyl group being selected from: CO―C₁₋₂₀ alkyl, CO―C₂₋₂₀ alkenyl, CO―C₅₋₁₀ aryl and CO―C₅₋₁₀ ar―C₁₋₂₀ alkyl | | | | | | | | | | | | |

e) at positions 30 and 31, it has:
(i) the Met at position 30 replaced by an alternative amino acid selected from Leu, NLeu, Ile, Val, Phe, Thr, Asn, Cys and Lys; and/or
(ii) the Leu at position 31 replaced by an alternative amino acid selected from Ile and Val
f) at positions 34, 35 and 36, it has:
(i) the Pro at position 34 replaced by the alternative amino acid Gln, His or NVa (Nor-valine); and/or
(ii) the Arg at position 3 5 replaced by the alternative amino acid Gln or HArg; and/or
(iii) the Tyr at position 36 replaced by the alternative amino acid Phe
g) it is dimerised by disulphide bond formation between a pair of Cys residues added to the two units at the C termini or as replacements for Leu at position 31 in the two units, whereby when the analogue has Ala1 absent then it has at least one further change other than one or more selected from Nle17 and Nle30,
whereby when the analogue has one or more of N1e17, N1e30 and His34, then it has at least one further change other than the one or more selected from N1e17, N1e30 and His34, and
whereby when the analogue has Gln at position 34, then it has at least one further change other than one or more selected from Ile31, Val31, Leu30 and NLeu30,

Optionally, if the analogue has one or more of:
- Arg at position 19 (optionally with Phe at position 6)
- Tyr or Glu at position 21 (optionally with Phe at position 6)
- Ser at position 22 (optionally with Phe at position 6)
- Ala or Gln at position 23 (optionally with Phe at position 6)
or if the analogue has one of:
- Glu at position 23 and Glu at position 6
- Asp at position 11 and Gln at position 15 and Glu at position 23 and Met at position 24
- Met at position 6 and Tyr at position 11 and Glu at position 21 and Thr at position 22 and
- Gln at position 23 and Thr at position 30
then the analogue has at least one further difference from the native human Pancreatic Polypeptide.

The analogues may optionally be for use (i) in the treatment (including prophylactic treatment) of obesity or diabetes in a subject, or (ii) in the reduction of appetite in a subject, the reduction of food intake in a subject, or in the reduction of calorie intake in a subject.

### Variants:

The remaining portions of the PP molecule may be variants of the native portions, for example they may be variants of the equivalent native human portions. Variants include PP portions with deletions, insertions, inversions, repeats and substitutions, (e.g., conservative substitutions and non-conservative substitutions; see, e.g., Table 1 below) which retain at least some of the activity of a corresponding non-variant molecule when in a molecule of the invention. More than one amino acid (e.g., 2, 3 or 4) can be deleted or inserted or substituted with another amino acid. Accordingly, the PP portions as present in a compound of formula (I) preferably include at least 25 amino acids of the native sequence of human PP, more preferably at least 28 amino acids of the native sequence of human PP, for example at least 30 amino acids of the native sequence of human PP, for example at least 32 or at least 34 amino acids of the native sequence of human PP.

For example, the compound of formula (I) preferably includes at least 25 amino acids of the native sequence of human PP, more preferably at least 28 amino acids of the native sequence of human PP, for example at least 30 amino acids of the native sequence of human PP, for example at least 32 or at least 34 amino acids of the native sequence of human PP.

Typically conservative substitutions are the replacements, for one another, among the aliphatic amino acids Ala, Val, Leu and Ile; interchange of Ser and Thr containing hydroxy residues, interchange of the acidic residues Asp and Glu, interchange between the amide residues Asn and Gln, interchange of the basic residues Lys and Arg, interchange of the aromatic residues Phe and Tyr, and interchange of the small-sized amino acids Ala, Ser, Thr, Met and Gly. Guidance concerning how to make phenotypically silent amino acid substitutions, ie substitutions that do not alter the expressed phenotype, is provided in Bowie et al., Science 247:1306-1310, 1990.

**Table 1: Non-limiting examples of conservative amino acid substitutions**

| Original Residue | Conservative Substitutions |
|---|---|
| | |
| Ala | Ser |
| Arg | Lys |
| Asn | Gln, His |
| Asp | Glu |
| Cys | Ser |
| Gln | Asn |
| Glu | Asp |
| His | Asn; Gln |
| Ile | Leu, Val |
| Leu | Ile; Val |
| Lys | Arg; Gln |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

A further example of a variant is a compound with Glu at position 4 replaced with Lys. In one embodiment, variants do not include compounds with Glu at position 4 replaced with Lys. A further example of a variant is a compound with Ile at position 28 replaced with Leu. A further example of a variant is a compound with Arg at position 33 replaced with Lys.

Variants of PP further include variants in which one or more amino acids (for example 2, 3 or 4) of the PP of one species are substituted by an amino acid present at the equivalent position in the PP derived from a different species. The sequences of the PPs of various species are known.

In one embodiment, the analogue is not a variant.

### Derivatives

A compound of the invention may comprise the structure of formula (I) modified by well known processes including amidation, glycosylation, carbamylation, acylation, for example acetylation, sulfation, phosphylation, cyclization, lipidization and pegylation. The structure of formula (I) may be modified at random positions within the molecule, or at predetermined positions within the molecule and may include one, two, three or more attached chemical moieties. Derivatives include compounds in which the N-terminal NH2 group is replaced with another group, for example a methoxy group.

A compound of the invention may be a fusion protein, whereby the structure of formula (I) is fused to another protein or polypeptide (the fusion partner) using recombinant methods known in the art. Alternatively, such a fusion protein may be synthetically synthesized by any known method. Such a fusion protein comprises the structure of formula (I). Any suitable peptide or protein can be used as the fusion partner (e.g., serum albumin, carbonic anhydrase, glutathione-S-transferase or thioredoxin, etc.). Preferred fusion partners will not have an adverse biological activity *in vivo.* Such fusion proteins may be made by linking the carboxy-terminus of the fusion partner to the amino-tenninus of the structure of formula (I) or vice versa. Optionally, a cleavable linker may be used to link the structure of formula (I) to the fusion partner. A resulting cleavable fusion protein may be cleaved *in vivo* such that an active form of a compound of the invention is released. Examples of such cleavable linkers include, but are not limited to, the linkers D-D-D-D-Y, G-P-R, A-G-G and H-P-F-H-L, which can be cleaved by enterokinase, thrombin, ubiquitin cleaving enzyme and renin, respectively. See, e.g., U.S. Patent No. 6,410,707.

A compound of the invention may be a physiologically functional derivative of the structure of formula (I). The term "physiologically functional derivative" is used herein to denote a chemical derivative of a compound of formula (I) having the same physiological function as the corresponding unmodified compound of formula (I). For example, a physiologically functionally derivative may be convertible in the body to a compound of formula (I). According to the present invention, examples of physiologically functional derivatives include esters, amides, and carbamates; preferably esters and amides.

Pharmaceutically acceptable esters and amides of the compounds of the invention may comprise a C₁₋₂₀ alkyl-, C₂₋₂₀ alkenyl-, C₅₋₁₀ aryl-, C₅-₁₀ ar-C₁₋₂₀ alkyl-, or amino acid-ester or -amide attached at an appropriate site, for example at an acid group. Examples of suitable moieties are hydrophobic substituents with 4 to 26 carbon atoms, preferably 5 to 19 carbon atoms. Suitable lipid groups include, but are not limited to, the following: lauroyl (C₁₂H₂₃), palmityl (C₁₅H₃₁), oleyl (C₁₅H₂₉), stearyl (C₁₇H₃₅), cholate; and deoxycholate.

Methods for lipidization of sulfhydryl-containing compounds with fatty acid derivatives are disclosed in U.S. Patent No. 5,936,092; U.S. Patent No. 6,093,692; and U.S. Patent No. 6,225,445. Fatty acid derivatives of a compound of the invention comprising a compound of the invention linked to fatty acid via a disulfide linkage may be used for delivery of a compound of the invention to neuronal cells and tissues. Lipidisation markedly increases the absorption of the compounds relative to the rate of absorption of the corresponding unlipidised compounds, as well as prolonging blood and tissue retention of the compounds. Moreover, the disulfide linkage in lipidised derivative is relatively labile in the cells and thus facilitates intracellular release of the molecule from the fatty acid moieties. Suitable lipid-containing moieties are hydrophobic substituents with 4 to 26 carbon atoms, preferably 5 to 19 carbon atoms. Suitable lipid groups include, but are not limited to, the following: palmityl (C₁₅H₃₁,), oleyl (C₁₅H₂₉), stearyl (C₁₇H₃₅), cholate; and deoxycholate.

Cyclization methods include cyclization through the formation of a disulfide bridge and head-to-tail cyclization using a cyclization resin. Cyclized peptides may have enhanced stability, including increased resistance to enzymatic degradation, as a result of their conformational constraints. Cyclization may in particular be expedient where the uncyclized peptide includes an N-terminal cysteine group. Suitable cyclized peptides include monomeric and dimeric head-to-tail cyclized structures. Cyclized peptides may include one or more additional residues, especially an additional cysteine incorporated for the purpose of formation of a disulfide bond or a side chain incorporated for the purpose of resin-based cyclization.

A compound of the invention may be a pegylated structure of formula (I). Pegylated compounds of the invention may provide additional advantages such as increased solubility, stability and circulating time of the polypeptide, or decreased immunogenicity (see U.S. Patent No. 4,179,337).

Chemical moieties for derivitization of a compound of the invention may also be selected from water soluble polymers such as polyethylene glycol, ethylene glycol/propylene glycol copolymers, carboxymethylcellulose, dextran, polyvinyl alcohol and the like. A polymer moiety for derivatisation of a compound of the invention may be of any molecular weight, and may be branched or unbranched. For ease in handling and manufacturing, the preferred molecular weight of a polyethylene glycol for derivatisation of a compound of the invention is from about 1 kDa to about 100 kDa, the term "about" indicating that in preparations of polyethylene glycol, some molecules will weigh more, some less, than the stated molecular weight. Polymers of other molecular weights may be used, depending on the desired therapeutic profile, for example the duration of sustained release desired, the effects, if any on biological activity, the ease in handling, the degree or lack of antigenicity and other known effects of the polyethylene glycol to a therapeutic protein or analog. For example, the polyethylene glycol may have an average molecular weight of about 200, 500, 1000, 1500, 2000, 2500, 3000, 3500, 4000, 4500, 5000, 5500, 6000, 6500, 7000, 7500, 8000, 8500, 9000, 9500,10,000,10,500,11,000,11,500,12,000, 12,500,13,000, 13,500, 14,000, 14,500; 15,000,15,500,16,000,16,500, 17,000 , 17,500, 18,000, 18,500, 19,000,19,500,20,000,25,000, 30,000,35,000,40,000, 50,000, 55,000, 60,000, 65,000, 70,000, 75,000, 80,000, 85,000, 90,000, 95,000, or 100,000 kDa.

Salts and solvates of compounds of the invention that are suitable for use in a medicament are those wherein a counterion or associated solvent is pharmaceutically acceptable. However, salts and solvates having non-phannaceutically acceptable counterions or associated solvents are within the scope of the present invention, for example, for use as intermediates in the preparation of the compounds of formula (I) and their pharmaceutically acceptable salts or solvates.

Suitable salts according to the invention include those formed with organic or inorganic acids or bases. Pharmaceutically acceptable acid addition salts include those formed with hydrochloric, hydrobromic, sulphuric, nitric, citric, tartaric, acetic, phosphoric, lactic, pyruvic, acetic, trifluoroacetic, succinic, perchloric, fumaric, maleic, glycollic, lactic, salicylic, oxaloacetic, methanesulfonic, ethanesulfonic, p-toluenesulfonic, formic, benzoic, malonic, naphthalene-2-sulfonic, benzenesulfonic, and isethionic acids. Other acids such as oxalic, while not in themselves pharmaceutically acceptable, may be useful as intermediate in obtaining the compounds of the invention and their pharmaceutical acceptable salts. Pharmaceutically acceptable salts with bases include ammonium salts, alkali metal salts, for example potassium and sodium salts, alkaline earth metal salts, for example calcium and magnesium salts, and salts with organic bases, for example dicyclohexylamine and N-methyl-D-glucomine.

Those skilled in the art of organic chemistry will appreciate that many organic compounds can form complexes with solvents in which they are reacted or from which they are precipitated or crystallized. Such complexes are known as "solvates". For example, a complex with water is known as a "hydrate". The present invention provides solvates of compounds of the invention.

### Conditions:

The invention provides a pharmaceutical composition comprising a compound of formula (I). The invention further provides the compound of formula (I) for use as a medicament.

The invention also provides a compound of formula (I) for use in the treatment (including prophylactic treatment) of obesity or diabetes. The invention further provides a compound of formula (I) for use in reduction of appetite in a subject, for use in reduction of food intake in a subject, or for use in reduction of calorie intake in a subject.

The invention further provides the use of a compound of formula (I) for the manufacture of a medicament for the treatment (including prophylactic treatment) of obesity or diabetes. The invention also provides the use of a compound of formula (I) for the manufacture of a medicament for reducing appetite in a subject, reducing food intake in a subject, or reducing calorie intake in a subject.

The invention further provides a method of treating (including prophylactic treatment) obesity or diabetes in a subject in need thereof comprising administering to the subject an effective amount of a compound of formula (I). The invention also provides a method of reducing appetite in a subject, reducing food intake in a subject, or reducing calorie intake in a subject, comprising administering to the subject an effective amount of a compound of formula (I).

The subject to whom the compound is administered may be overweight, for example, obese. Alternatively, or in addition, the subject may be diabetic, for example having insulin resistance or glucose intolerance, or both. The subject may have diabetes mellitus, for example, the subject may have Type II diabetes. The subject may be overweight, for example, obese and have diabetes mellitus, for example, Type II diabetes.

In addition, or alternatively, the subject may have, or may be at risk of having, a disorder in which obesity or being overweight is a risk factor. Such disorders include, but are not limited to, cardiovascular disease, for example hypertension, atherosclerosis, congestive heart failure, and dyslipidemia; stroke; gallbladder disease; osteoarthritis; sleep apnea; reproductive disorders for example, polycystic ovarian syndrome; cancers, for example breast, prostate, colon, endometrial, kidney, and esophagus cancer; varicose veins; acnthosis nigricans; eczema; exercise intolerance; insulin resistance; hypertension hypercholesterolemia; cholithiasis; osteoarthritis; orthopedic injury; insulin resistance, for example, type 2 diabetes and syndrome X; and thromboembolic disease (see Kopelman, Nature 404:635-43; Rissanen et al., British Med. J. 301, 835, 1990).

Other disorders associated with obesity include depression, anxiety, panic attacks, migraine headaches, PMS, chronic pain states, fibromyalgia, insomnia, impulsivity, obsessive compulsive disorder, and myoclonus. Furthermore, obesity is a recognized risk factor for increased incidence of complications of general anesthesia. (See e. g., Kopelman, Nature 404:635-43, 2000). In general, obesity reduces life span and carries a serious risk of co-morbidities such as those listed above.

Other diseases or disorders associated with obesity are birth defects, maternal obesity being associated with increased incidence of neural tube defects, carpal tunnel syndrome (CTS); chronic venous insufficiency (CVI); daytime sleepiness; deep vein thrombosis (DVT); end stage renal disease (ESRD); gout; heat disorders; impaired immune response; impaired respiratory function; infertility; liver disease; lower back pain; obstetric and gynecologic complications; pancreatititis; as well as abdominal hernias; acanthosis nigricans; endocrine abnormalities; chronic hypoxia and hypercapnia; dermatological effects; elephantitis; gastroesophageal reflux; heel spurs; lower extremity edema; mammegaly which causes considerable problems such as bra strap pain, skin damage, cervical pain, chronic odors and infections in the skin folds under the breasts, etc.; large anterior abdominal wall masses, for example abdominal panniculitis with frequent panniculitis, impeding walking, causing frequent infections, odors, clothing difficulties, low back pain; musculoskeletal disease; pseudo tumor cerebri (or benign intracranial hypertension), and sliding hiatil hernia.

The present invention further provides a method for increasing energy expenditure in a subject. The method includes, for example, peripherally administering a therapeutically effective amount of a compound of the invention to the subject, thereby altering energy expenditure. Energy is burned in all physiological processes. The body can alter the rate of energy expenditure directly, by modulating the efficiency of those processes, or changing the number and nature of processes that are occurring. For example, during digestion the body expends energy moving food through the bowel, and digesting food, and within cells, the efficiency of cellular metabolism can be altered to produce more or less heat.

In one aspect, the method of the invention involves manipulation of the arcuate circuitry, that alter food intake coordinately and reciprocally alter energy expenditure. Energy expenditure is a result of cellular metabolism, protein synthesis, metabolic rate, and calorie utilization. Thus, in this aspect of the invention, administration of a compound of formula (1) results in increased energy expenditure, and decreased efficiency of calorie utilization.

The invention also provides a method for improving a lipid profile in a subject. The invention also provides a method for alleviating a condition or disorder that can be alleviated by reducing nutrient availability.

Appetite can be measured by any means known to one of skill in the art. For example, decreased appetite can be assessed by a psychological assessment. For example, administration of a compound of the invention results in a change in perceived hunger, satiety, and/or fullness. Hunger can be assessed by any means known to one of skill in the art. For example, hunger is assessed using psychological assays, such as by an assessment of hunger feelings and sensory perception using a questionnaire, such as, but not limited to, a Visual Analog Score (VAS) questionnaire. In one specific, non-limiting example, hunger is assessed by answering questions relating to desire for food, drink, prospective food consumption, nausea, and perceptions relating to smell or taste.

A compound of the invention may be used for weight control and treatment, for example reduction or prevention of obesity, in particular any one or more of the following: preventing and reducing weight gain; inducing and promoting weight loss; and reducing obesity as measured by the Body Mass Index. A compound of the invention may be used in the control of any one or more of appetite, satiety and hunger, in particular any one or more of the following: reducing, suppressing and inhibiting appetite; inducing, increasing, enhancing and promoting satiety and sensations of satiety; and reducing, inhibiting and suppressing hunger and sensations of hunger. A compound of the invention may be used in maintaining any one or more of a desired body weight, a desired Body Mass Index, a desired appearance and good health.

A subject may be a subject who desires weight loss, for example female and male subjects who desire a change in their appearance. A subject may desire decreased feelings of hunger, for example the subject may be a person involved in a lengthy task that requires a high level of concentration, for example soldiers on active duty, air traffic controllers, or truck drivers on long distance routes, etc.

The present invention may also be used in treating, prevention, ameliorating or alleviating conditions or disorders caused by, complicated by, or aggravated by a relatively high nutrient availability. The term "condition or disorder which can be alleviated by reducing caloric (or nutrient) availability" is used herein to denote any condition or disorder in a subject that is either caused by, complicated by, or aggravated by a relatively high nutrient availability, or that can be alleviated by reducing nutrient availability, for example by decreasing food intake. Subjects who are insulin resistant, glucose intolerant, or have any form of diabetes mellitus, for example, type 1, 2 or gestational diabetes, can also benefit from methods in accordance with the present invention.

Conditions or disorders associated with increased caloric intake include, but are not limited to, insulin resistance, glucose intolerance, obesity, diabetes, including type 2 diabetes, eating disorders, insulin-resistance syndromes, and Alzheimer's disease.

According to the present invention, a compound of formula (I) is preferably used in the treatment of a human. However, while the compounds of the invention will typically be used to treat human subjects they may also be used to treat similar or identical conditions in other vertebrates for example other primates; farm animals for example swine, cattle and poultry; sport animals for example horses; companion animals for example dogs and cats.

### Compositions

While it is possible for the active ingredient to be administered alone, it is preferable for it to be present in a pharmaceutical formulation or composition. Accordingly, the invention provides a pharmaceutical formulation comprising a compound of formula (I), or a variant or derivative thereof, or a salt or solvate thereof, as defined above and a pharmaceutically acceptable excipient. Pharmaceutical compositions of the invention may take the form of a pharmaceutical formulation as described below.

The pharmaceutical formulations according to the invention include those suitable for oral, parenteral (including subcutaneous, intradermal, intramuscular, intravenous, and intraarticular), inhalation (including fine particle dusts or mists which may be generated by means of various types of metered does pressurized aerosols, nebulizers or insufflators), rectal and topical (including dermal, transdermal, transmucosal, buccal, sublingual, and intraocular) administration, although the most suitable route may depend upon, for example, the condition and disorder of the recipient.

The formulations may conveniently be presented in unit dosage form and may be prepared by any of the methods well known in the art of pharmacy. All methods include the step of bringing the active ingredient into association with the carrier which constitutes one or more accessory ingredients. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste. Various pharmaceutically acceptable carriers and their formulation are described in standard formulation treatises, e.g., Remington's Pharmaceutical Sciences by E. W. Martin. See also Wang, Y. J. and Hanson, M. A., Journal of Parenteral Science and Technology, Technical Report No. 10, Supp. 42:2S, 1988.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein. The present compounds can, for example, be administered in a form suitable for immediate release or extended release. Immediate release or extended release can be achieved by the use of suitable pharmaceutical compositions comprising the present compounds, or, particularly in the case of extended release, by the use of devices such as subcutaneous implants or osmotic pumps. The present compounds can also be administered liposomally.

Exemplary compositions for oral administration include suspensions which can contain, for example, microcrystalline cellulose for imparting bulk, alginic acid or sodium alginate as a suspending agent, methylcellulose as a viscosity enhance, and sweeteners or flavoring agents such as those known in the art; and immediate release tablets which can contain, for example, microcrystalline cellulose, dicalcium phosphate, starch, magnesium stearate and/or lactose and/or other excipients, binders, extenders, disintegrants, diluents and lubricants such as those known in the art. The compounds of formula (I) can also be delivered through the oral cavity by sublingual and/or buccal administration. Molded tablets, compressed tablets or freeze-dried tablets are exemplary forms which may be used. Exemplary compositions include those formulating the present compound(s) with fast dissolving diluents such as mannitol, lactose, sucrose and/or cyclodextrins. Also included in such formulations may be high molecular weight excipients such as celluloses (avicel) or polyethylene glycols (PEG). Such formulations can also include an excipient to aid mucosal adhesion such as hydroxy propyl cellulose (HPC), hydroxy propyl methyl cellulose (HPMC), sodium carboxy methyl cellulose (SCMC), maleic anhydride copolymer (e.g., Gantrez), and agents to control release such as polyacrylic copolymer (e.g. Carbopol 934). Lubricants, glidants, flavors, coloring agents and stabilizers may also be added for ease of fabrication and use.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example saline or water-for-injection, immediately prior to use. Extemporaneous injection solutions and suspensions may be prepared from sterile powders, granules and tablets of the kind previously described. Exemplary compositions for parenteral administration include injectable solutions or suspensions which can contain, for example, suitable non-toxic, parenterally acceptable diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid, or Cremaphor. An aqueous carrier may be, for example, an isotonic buffer solution at a pH of from about 3.0 to about 8.0, preferably at a pH of from about 3.5 to about 7.4, for example from 3.5 to 6.0, for example from 3.5 to about 5.0. Useful buffers include sodium citrate-citric acid and sodium phosphate-phosphoric acid, and sodium acetate/acetic acid buffers. The composition preferably does not include oxidizing agents and other compounds that are known to be deleterious to PP.
Excipients that can be included are, for instance, other proteins, such as human serum albumin or plasma preparations. If desired, the pharmaceutical composition may also contain minor amounts of non-toxic auxiliary substances, such as wetting or emulsifying agents, preservatives, and pH buffering agents and the like, for example sodium acetate or sorbitan monolaurate.

Exemplary compositions for nasal aerosol or inhalation administration include solutions in saline, which can contain, for example, benzyl alcohol or other suitable preservatives, absorption promoters to enhance bioavailability, and/or other solubilizing or dispersing agents such as those known in the art. Conveniently in compositions for nasal aerosol or inhalation administration the compound of the invention is delivered in the form of an aerosol spray presentation from a pressurized pack or a nebulizer, with the use of a suitable propellant, e.g., dichlorodifluoro-methane, trichlorofluoromethaie, dichlorotetrafluoroethane, carbon dioxide or other suitable gas. In the case of a pressurized aerosol the dosage unit can be determined by providing a valve to deliver a metered amount. Capsules and cartridges of e.g., gelatin for use in an inhaler or insufflator can be formulated to contain a powder mix of the compound and a suitable powder base, for example lactose or starch. In one specific, non-limiting example, a compound of the invention is administered as an aerosol from a metered dose valve, through an aerosol adapter also known as an actuator. Optionally, a stabilizer is also included, and/or porous particles for deep lung delivery are included (e.g., see U.S. Patent No. 6,447,743).

Formulations for rectal administration may be presented as a retention enema or a suppository with the usual carriers such as cocoa butter, synthetic glyceride esters or polyethylene glycol. Such carriers are typically solid at ordinary temperatures, but liquefy and/or dissolve in the rectal cavity to release the drug.

Formulations for topical administration in the mouth, for example buccally or sublingually, include lozenges comprising the active ingredient in a flavoured basis such as sucrose and acacia or tragacanth, and pastilles comprising the active ingredient in a basis such as gelatin and glycerine or sucrose and acacia. Exemplary compositions for topical administration include a topical carrier such as Plastibase (mineral oil gelled with polyethylene).

Preferred unit dosage formulations are those containing an effective dose, as hereinbefore recited, or an appropriate fraction thereof, of the active ingredient.

It should be understood that in addition to the ingredients particularly mentioned above, the formulations of this invention may include other agents conventional in the art having regard to the type of formulation in question, for example those suitable for oral administration may include flavouring agents.

The compounds of the invention are also suitably administered as sustained-release systems. Suitable examples of sustained-release systems of the invention include suitable polymeric materials, for example semi-permeable polymer matrices in the form of shaped articles, e.g., films, or mirocapsules; suitable hydrophobic materials, for example as an emulsion in an acceptable oil; or ion exchange resins; and sparingly soluble derivatives of the compound of the invention, for example, a sparingly soluble salt. Sustained-release systems may be administered orally; rectally; parenterally; intracisternally; intravaginally; intraperitoneally; topically, for example as a powder, ointment, gel, drop or transdermal patch; bucally; or as an oral or nasal spray.

Preparations for administration can be suitably formulated to give controlled release of compounds of the invention. For example, the pharmaceutical compositions may be in the form of particles comprising one or more of biodegradable polymers, polysaccharide jellifying and/or bioadhesive polymers, amphiphilic polymers, agents capable of modifying the interface properties of the particles of the compound of formula (I). These compositions exhibit certain biocompatibility features which allow a controlled release of the active substance. See U.S. Patent No. 5,700,486.

A compound of the invention may be delivered by way of a pump (see Langer, *supra;* Sefton, CRC Crit. Ref. Biomed. Eng. 14:201, 1987; Buchwald et al., Surgery 88:507, 1980; Saudek et al., N. Engl. J. Med. 321:574, 1989) or by a continuous subcutaneous infusions, for example, using a mini-pump. An intravenous bag solution may also be employed. The . key factor in selecting an appropriate dose is the result obtained, as measured by decreases in total body weight or ratio of fat to lean mass, or by other criteria for measuring control or prevention of obesity or prevention of obesity-related conditions, as are deemed appropriate by the practitioner. Other controlled release systems are discussed in the review by Langer (Science 249:1527-1533, 1990). In another aspect of the disclosure, compounds of the invention are delivered by way of an implanted pump, described, for example, in U.S. Patent No. 6,436,091; U.S. Patent No. 5,939,380; U.S. Patent No. 5,993,414.

Implantable drug infusion devices are used to provide patients with a constant and long term dosage or infusion of a drug or any other therapeutic agent. Essentially such device may be categorized as either active or passive. A compound of the present invention may be formulated as a depot preparation. Such a long acting depot formulation can be administered by implantation, for example subcutaneously or intramuscularly; or by intramuscular injection. Thus, for example, the compounds can be formulated with suitable polymeric or hydrophobic materials, for example as an emulsion in an acceptable oil; or ion exchange resins; or as a sparingly soluble derivatives, for example, as a sparingly soluble salt.

A therapeutically effective amount of a compound of the invention may be administered as a single pulse dose, as a bolus dose, or as pulse doses administered over time. Thus, in pulse doses, a bolus administration of a compound of the invention is provided, followed by a time period wherein no a compound of the invention is administered to the subject, followed by a second bolus administration. In specific, non-limiting examples, pulse doses of a compound of the invention are administered during the course of a day, during the course of a week, or during the course of a month.

In one embodiment, a therapeutically effective amount of a compound of the invention is administered with a therapeutically effective amount of another agent, for example an additional appetite suppressant, a food-intake-reducing, plasma glucose-lowering or plasma lipid-altering agent. Specific, non-limiting examples of an additional appetite suppressant include amfepramone (diethylpropion), phentermine, mazindol and phenylpropanolamine, fenfluramine, dexfenfluramine, and fluoxetine. The compound of the invention can be administered simultaneously with the additional appetite suppressant, or it may be administered sequentially. Thus, in one embodiment, the compound of the invention is formulated and administered with an appetite suppressant as a single dose.

A compound of the invention maybe administered whenever the effect, e.g., appetite suppression, decreased food intake, or decreased caloric intake, is desired, or slightly before to whenever the effect is desired, such as, but not limited to about 10 minutes, about 15 minutes, about 30 minutes, about 60 minutes, about 90 minutes, or about 120 minutes, before the time the effect is desired.

The therapeutically effective amount of a compound of the invention will be dependent on the molecule utilized, the subject being treated, the severity and type of the affliction, and the manner and route of administration. For example, a therapeutically effective amount of a compound of the invention may vary from about 0.01 µg per kilogram (kg) body weight to about 1 g per kg body weight, for example about 0.1 µg to about 20 mg per kg body weight, for example about 1 µg to about 5 mg per kg body weight, or about 5 µg to about 1 mg per kg body weight:

In one embodiment of the invention, a compound of the invention may be administered to a subject at from 5 to 1000 nmol per kg bodyweight, for example at from 10 to 750 nmol per kg bodyweight, for example at from 20 to 500 nmol per kg bodyweight, in particular at from 30 to 240 nmol per kg bodyweight. For a 75kg subject, such doses correspond to dosages of from 375 nmol to 75 µmol, for example from 750nmol to 56.25 µmol, for example from 1.5 to 37.5 µmol, in particular from 2.25 to 18 µmol.

In an alternative embodiment, a compound of the invention may be administered to a subject at 0.5 to 135 picomole (pmol) per kg body weight, for example 5 to 100 picomole (pmol) per kg body weight, for examples 10 to 90 picomole (pmol) per kg body weight, for example about 72 pmol per kg body weight. In one specific, non-limiting example, a compound of the invention is administered in a dose of about 1 nmol or more, 2 nmol or more, or 5 nmol or more. In this example, the dose of the compound of the invention is generally not more than 100 nmol, for example, the dose is 90 nmols or less, 80 nmols or less, 70 nmols or less, 60 nmols or less, 50 nmols or less, 40 nmols or less, 30 nmols or less, 20 nmols or less, 10 nmols. For example, a dosage range may comprise any combination of any of the specified lower dose limits with any of the specified upper dose limits. Thus, examples of non-limiting dose ranges of compounds of the invention are within the range of from 1 to 100 nmols, from 2 to 90 mols, from 5 to 80 nmol.

In one specific, non-limiting example, from about 1 to about 50 nmol of a compound of the invention is administered, for example about 2 to about 20 nmol, for example about 10 nmol is administered as a subcutaneous injection. The exact dose is readily determined by one of skill in the art based on the potency of the specific compound utilized, the route of delivery of the compound and the age, weight, sex and physiological condition of the subject.

Suitable doses of compounds of the invention also include those that result in a reduction in calorie intake, food intake, or appetite, or increase in energy expenditure that is equivalent to the reduction in calorie intake, food intake, or appetite, or to increase the energy expenditure, caused by the normal postprandial level of PP. Examples of doses include, but are not limited to doses that produce the effect demonstrated when the serum levels of PP are from 35 to 120 pmol/1, for example from 40 to 100 pmol/1, for example from 50 to 80pmol/1.

The doses of the compound may be administered once per day or more often. For example, they may be administered twice per day, three times per day or four times per day. For appetite suppression, three times daily administration is particularly advantageous as it corresponds to the usual three meals per eating pattern.

The disclosure is illustrated by the following non-limiting Examples.

### 8. EXAMPLES

### Peptide Synthesis:

Synthesis of the peptides of the invention was carried out on a tryclic amide linker resin with amino acids being attached using the Fmoc strategy. Each amino acid was added sequentially from the C- to the N-termini. Peptide couplings were mediated by the reagent TBTU. Peptide cleavage from the resin was achieved with trifluoracetic acid in the presence of scavengers. Synthesis using tryclic amide resin and Fmoc chemistry is also possible.

Peptides were purified by reverse phase HPLC. Peptides were shown to be greater than 90% pure by HPLC.

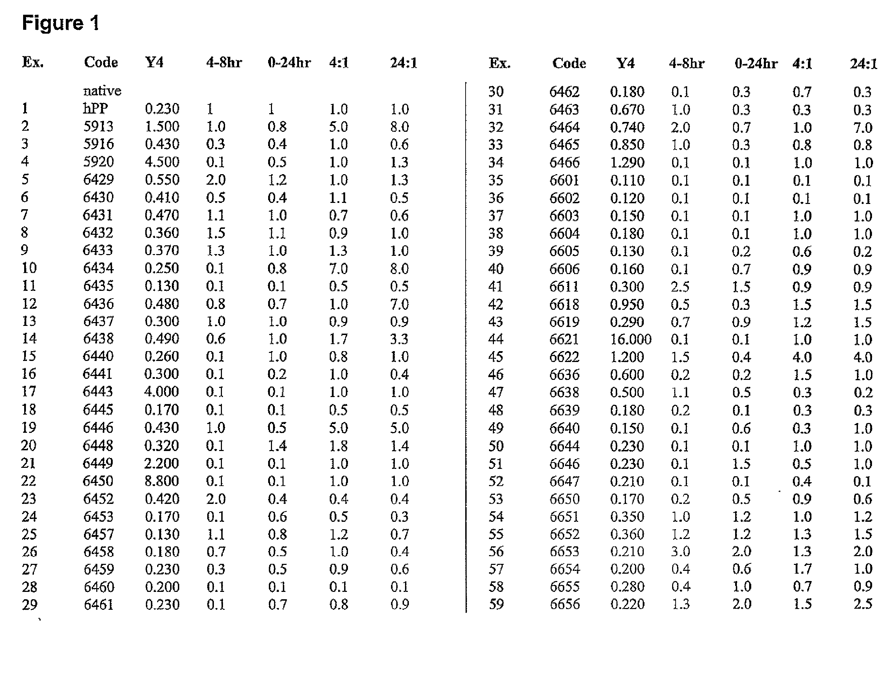

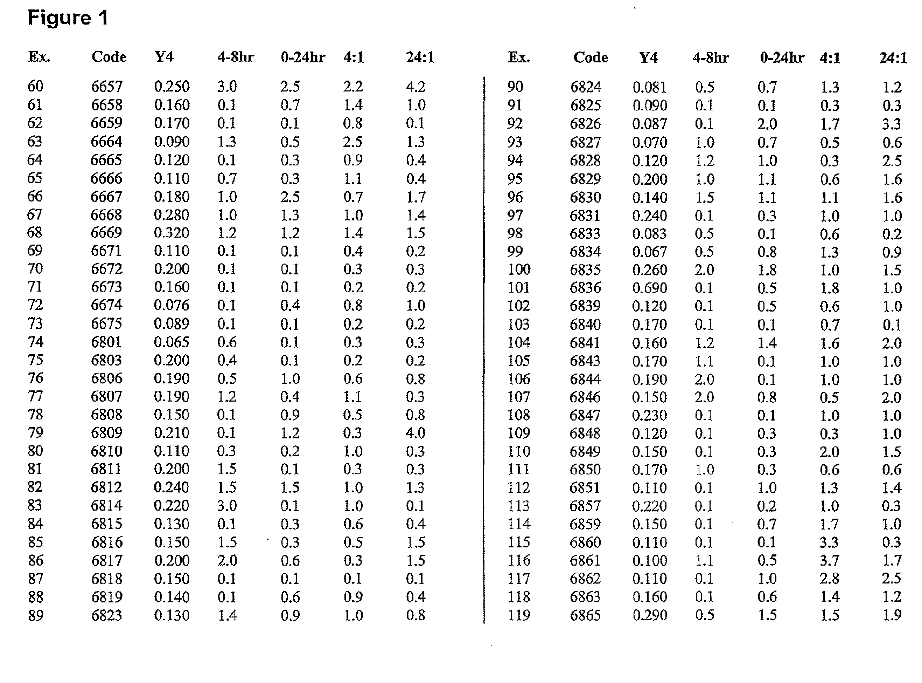

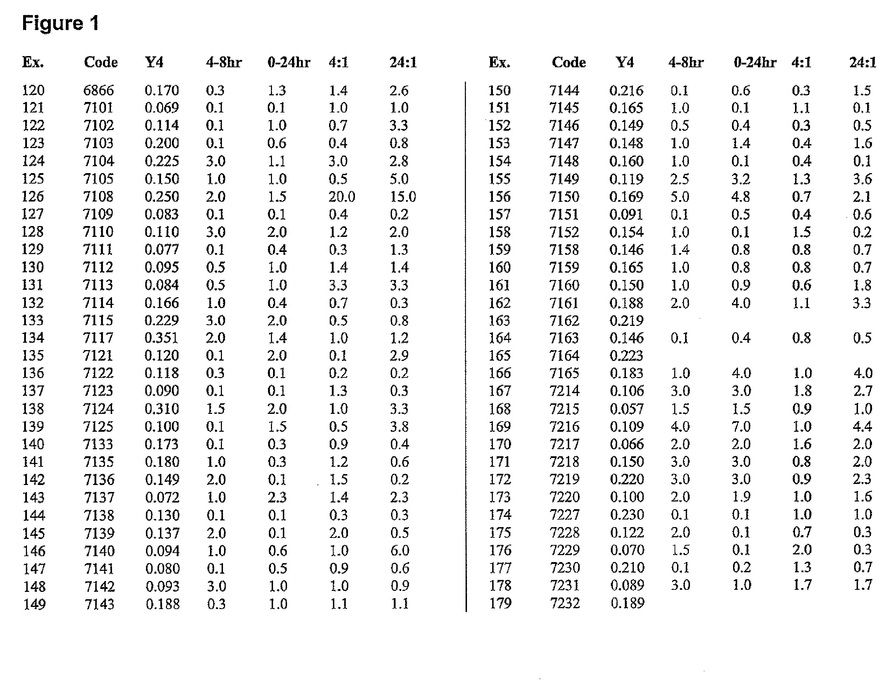

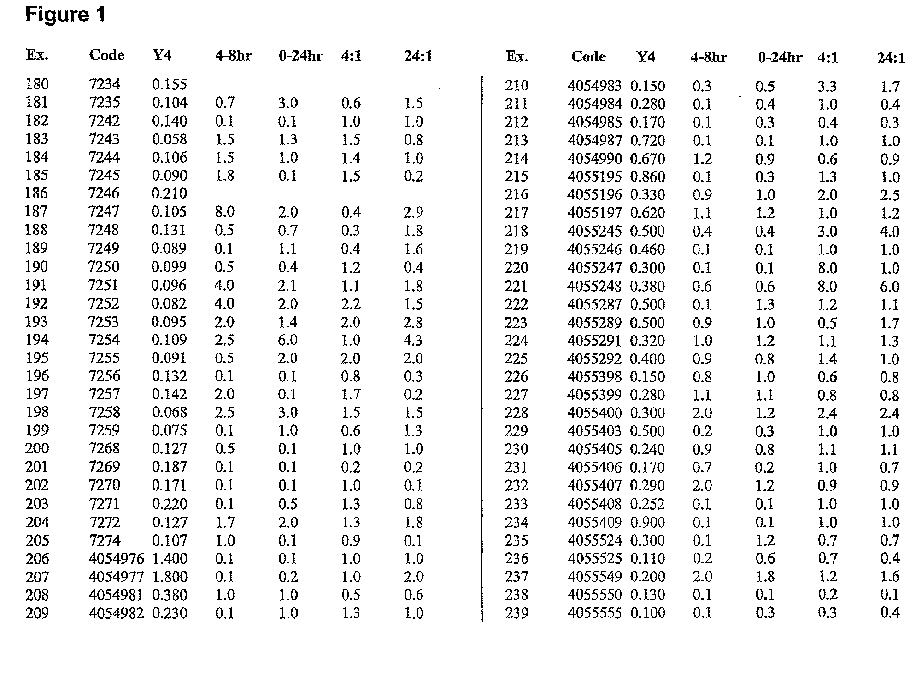

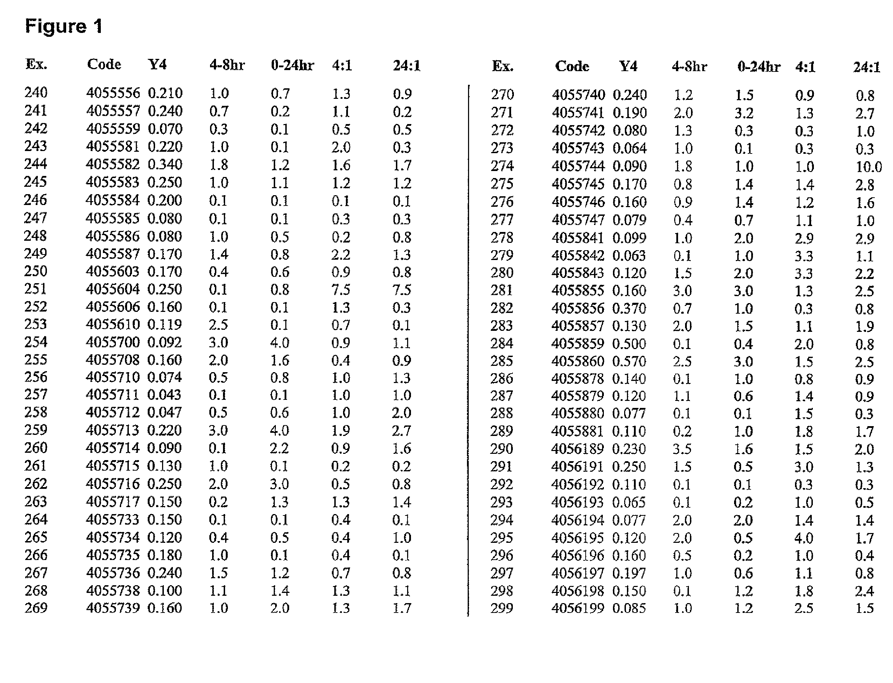

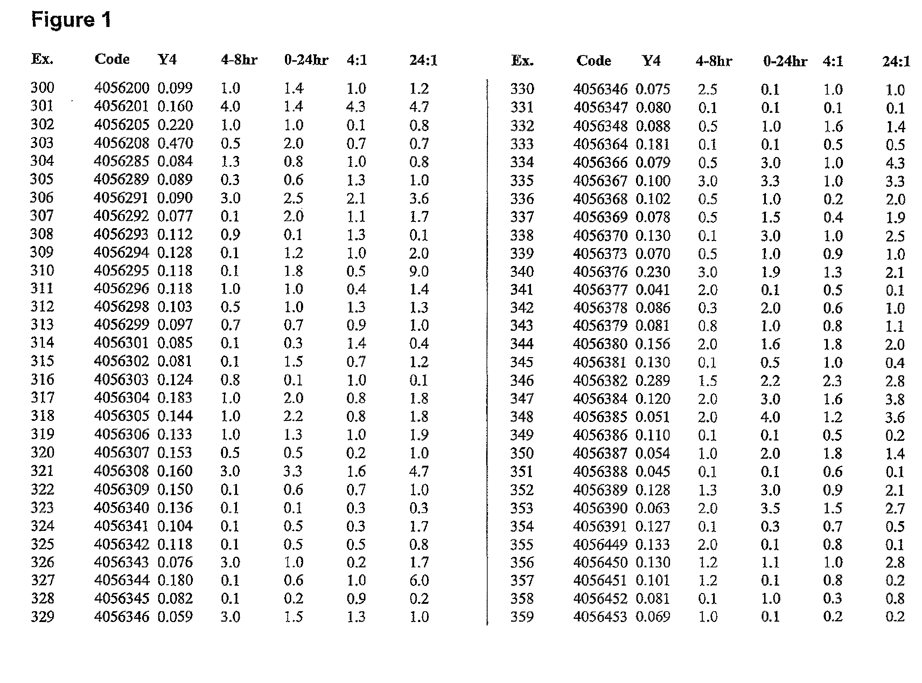

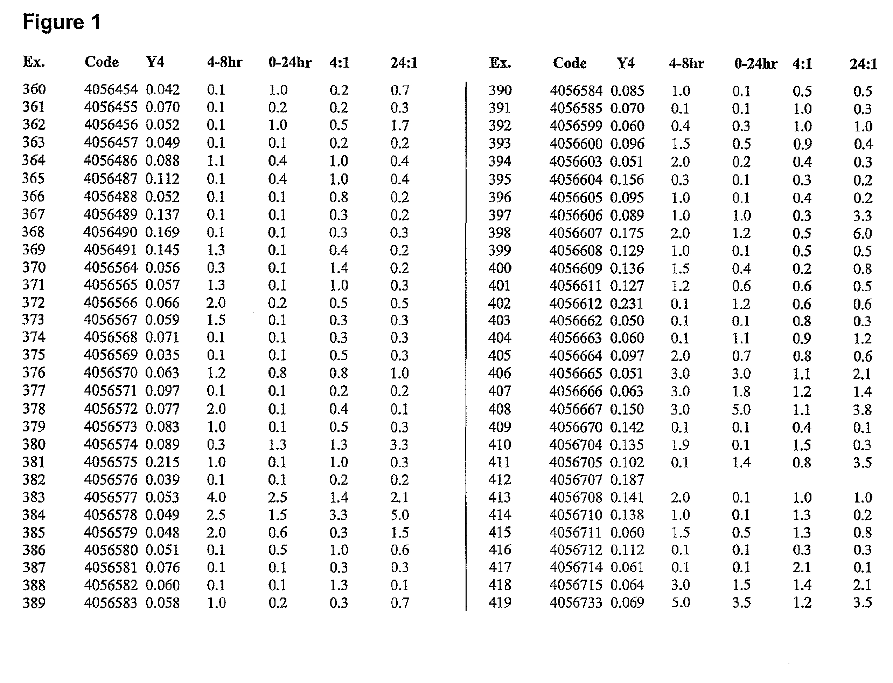

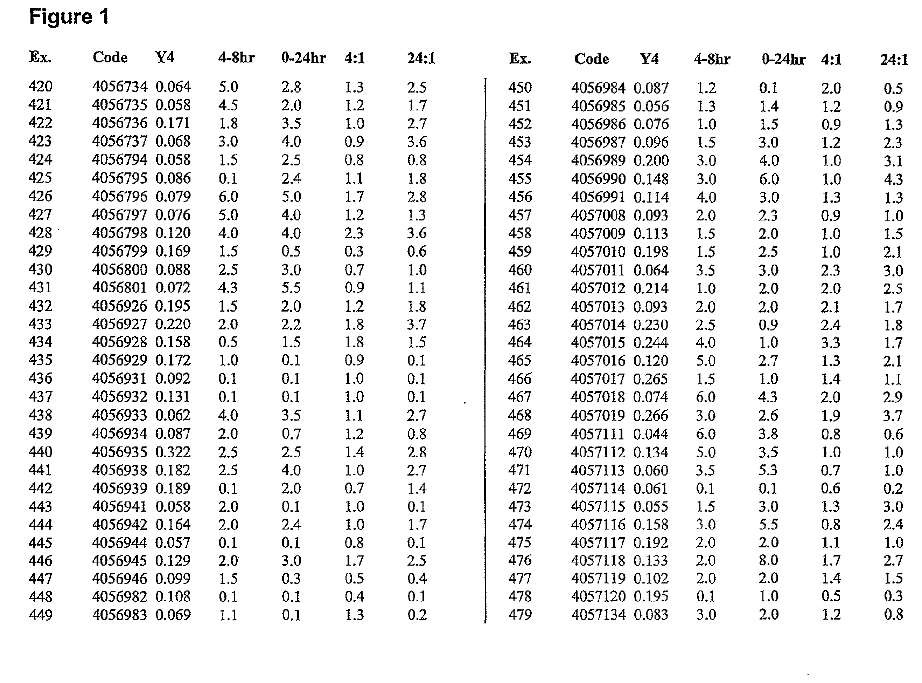

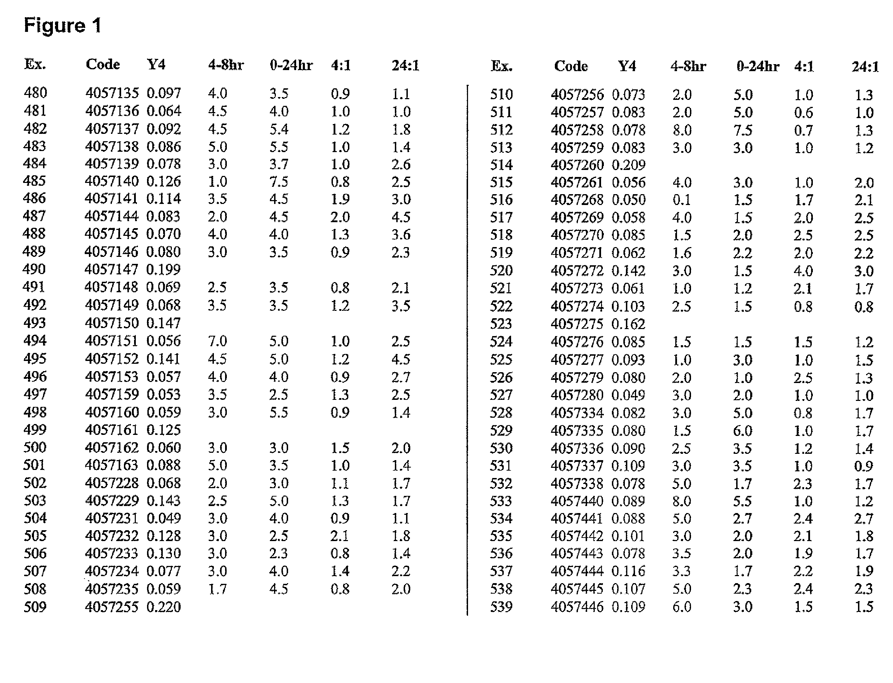

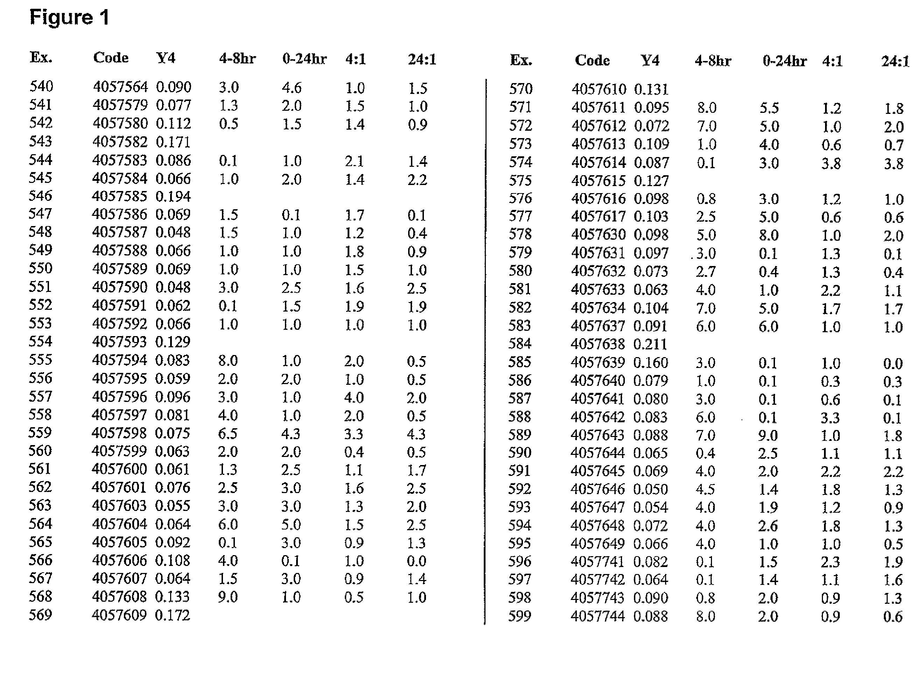

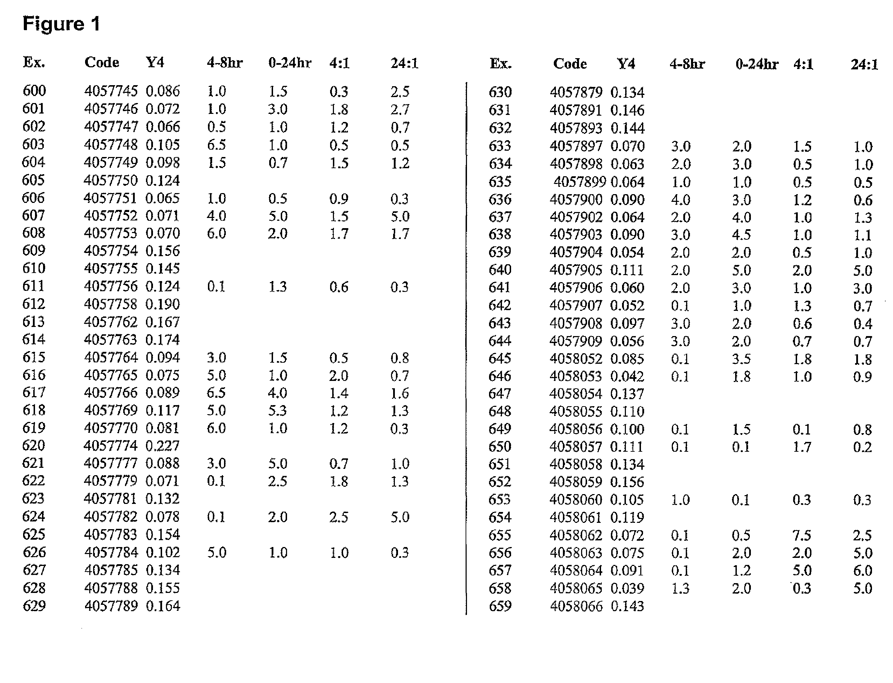

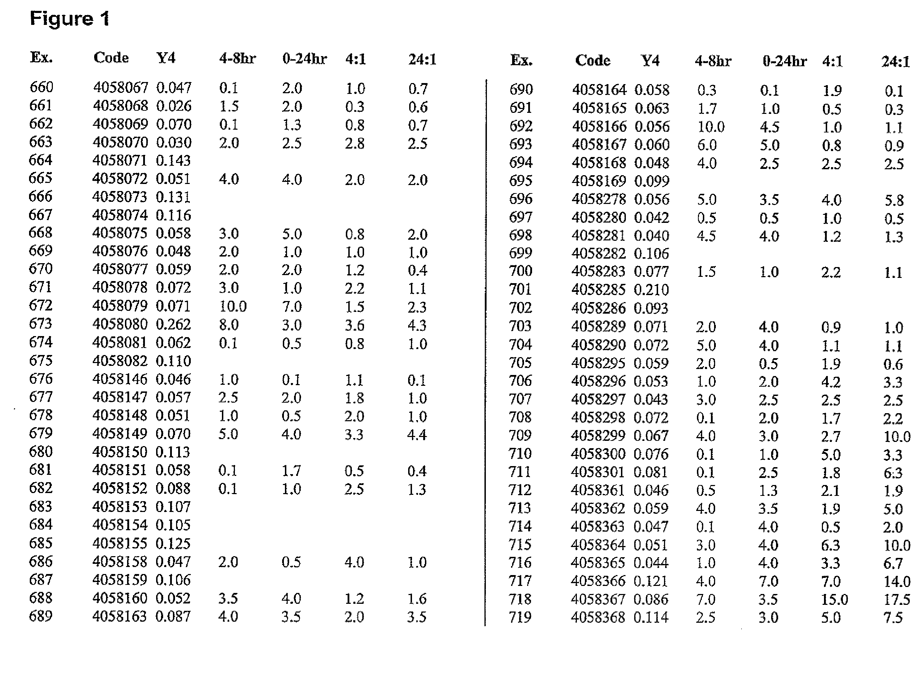

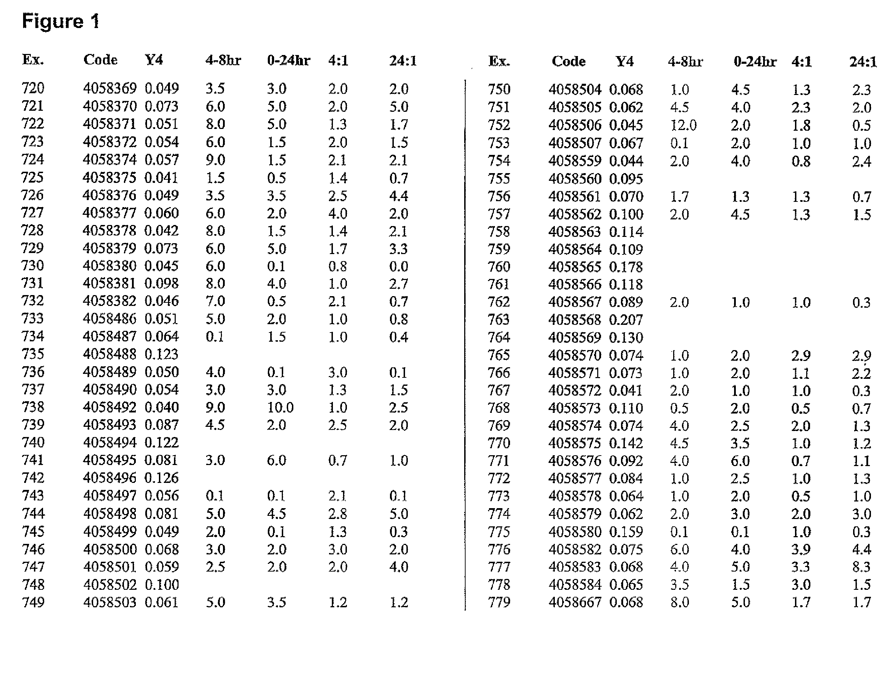

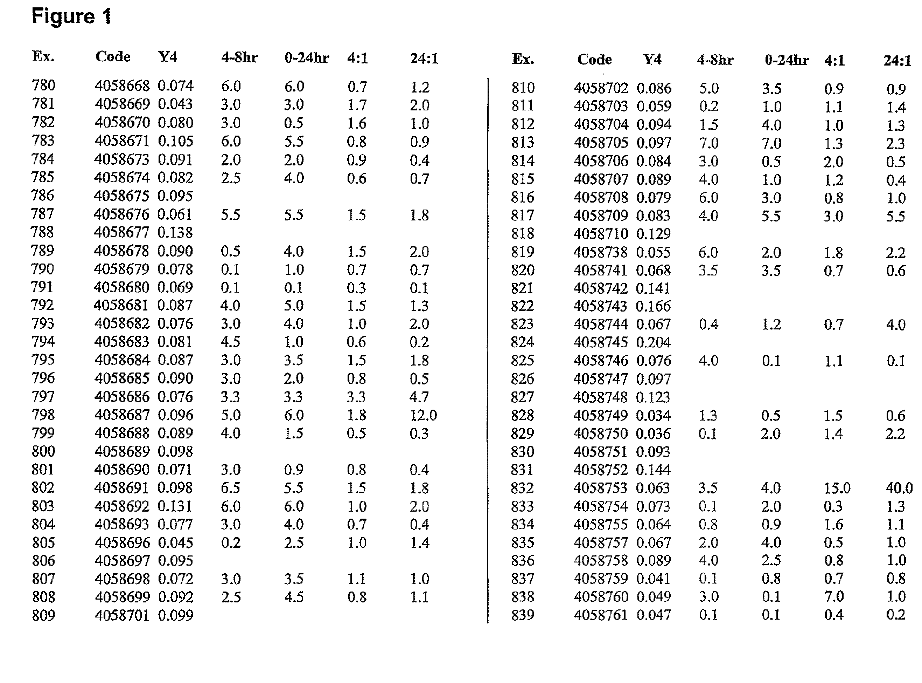

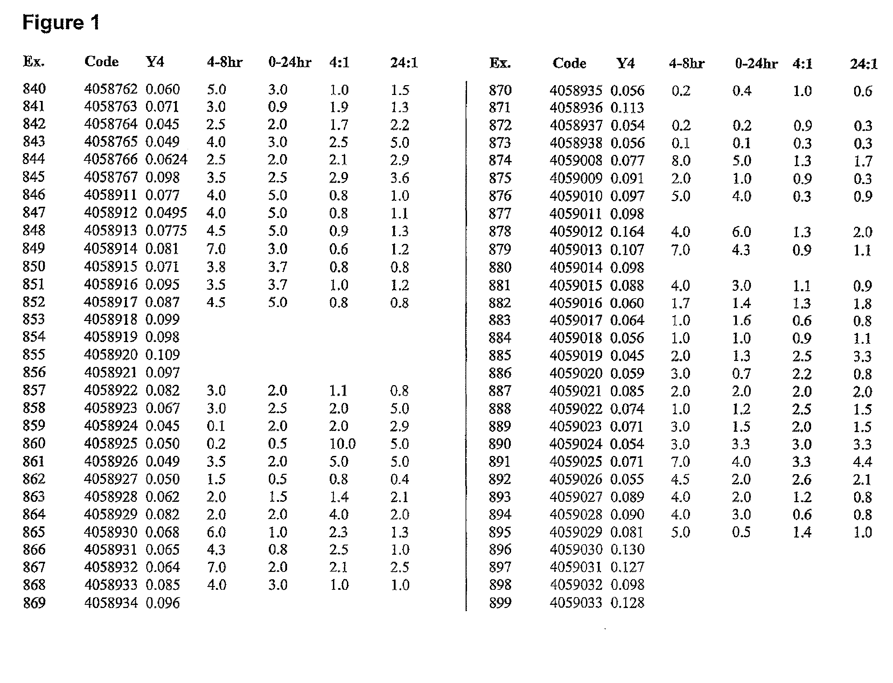

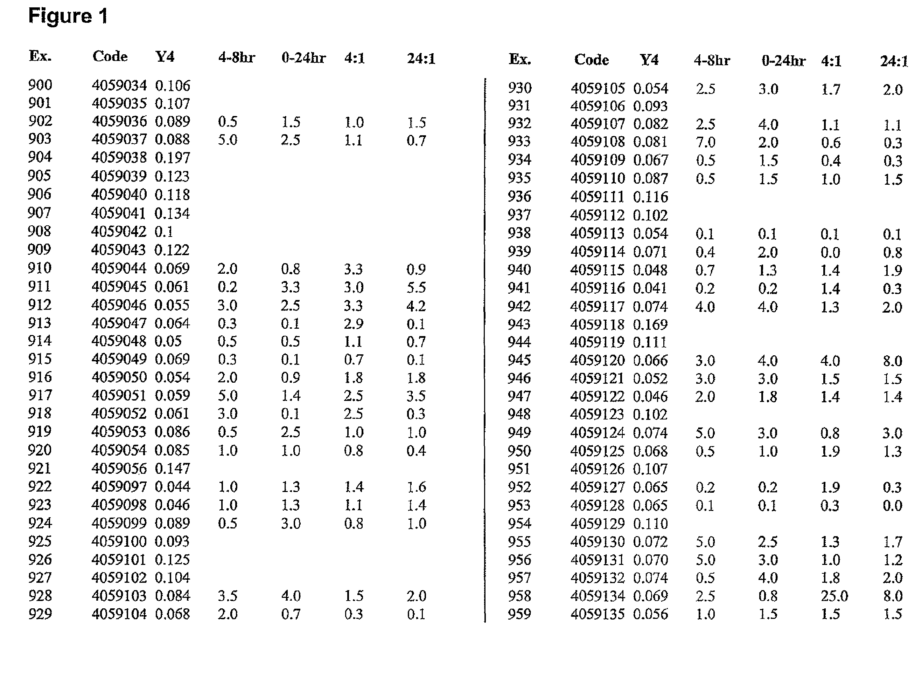

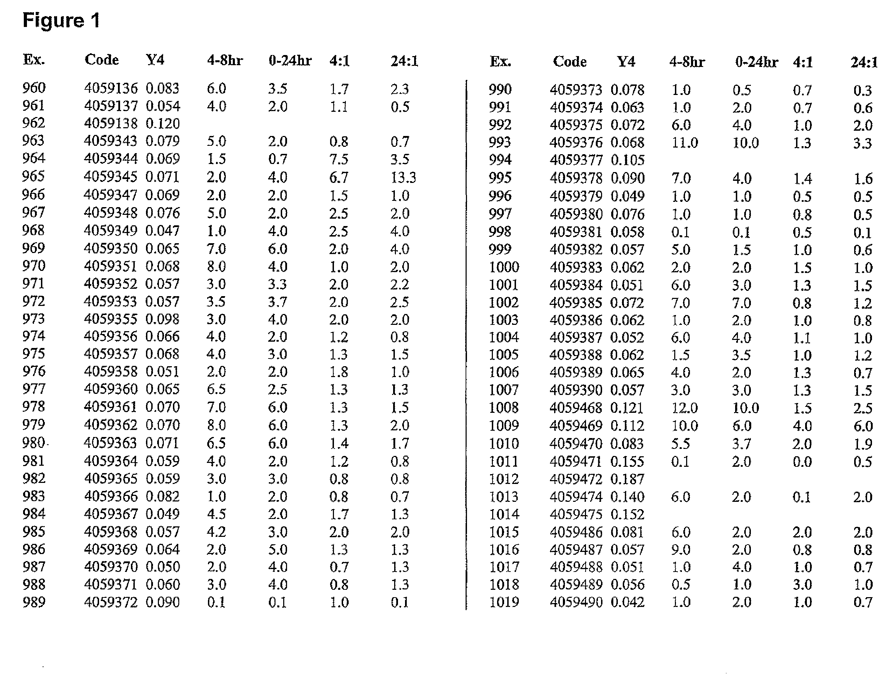

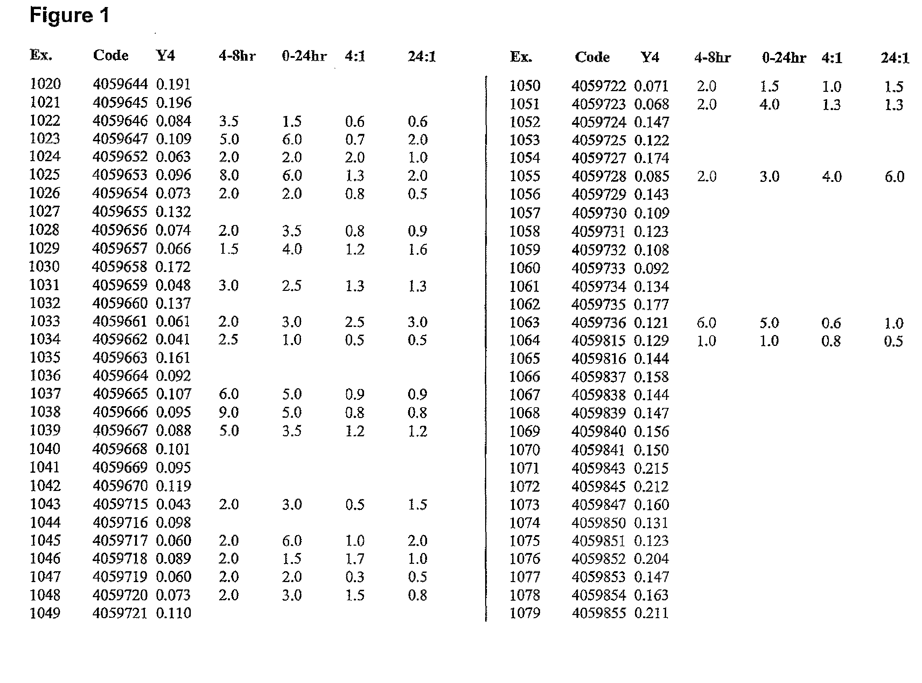

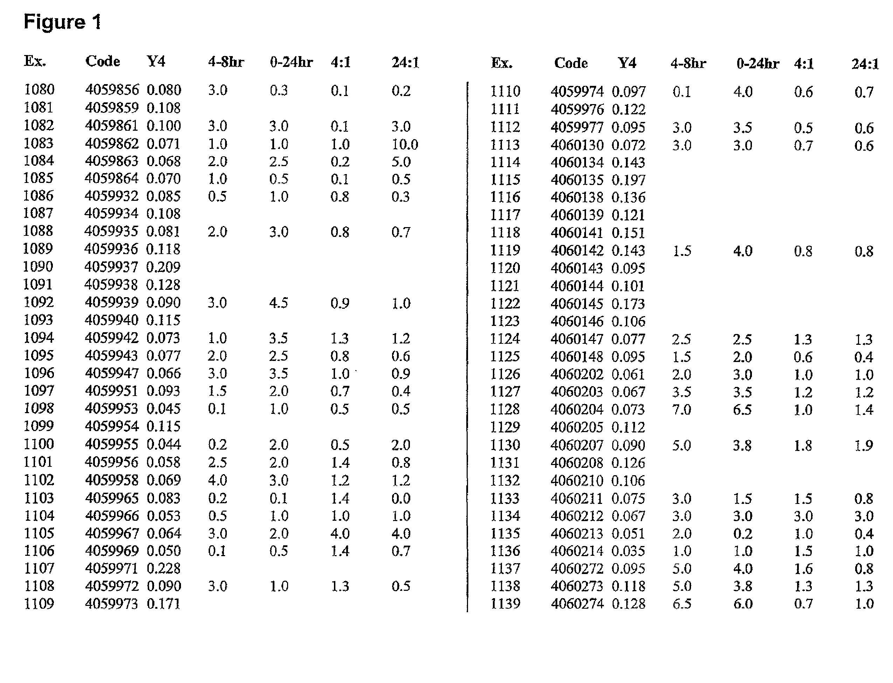

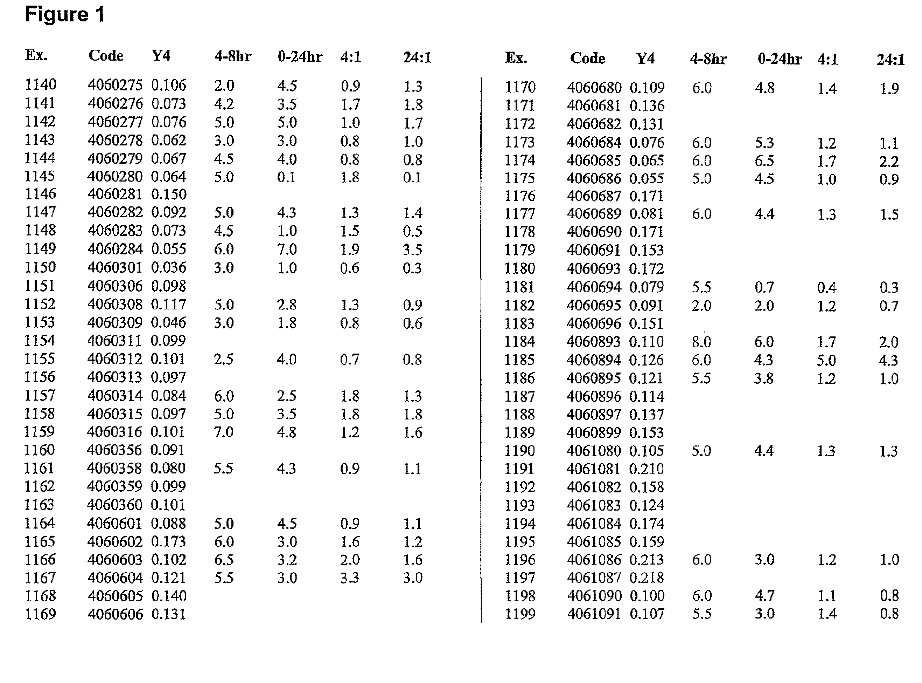

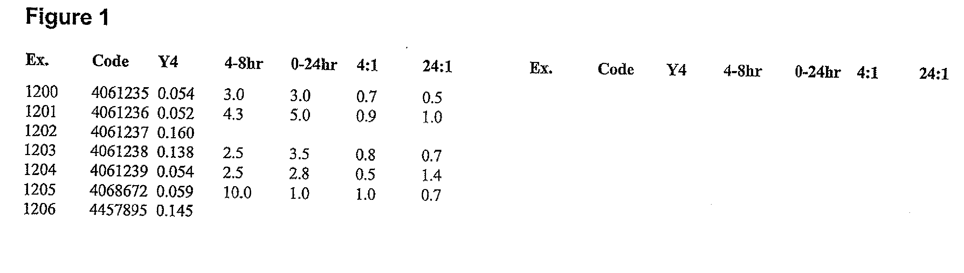

The peptides of example 1 to : 1206 have the sequences disclosed in the sequence listing as SEQ ID NO:1 to SEQ ID NO: 1206. Results of experiments using those sequences are given in the Table in Figure 1. Example 1 (which utilizes a peptide having the sequence in SEQ ID NO. 1 of the sequence listing) is a comparative Example illustrating the activity of of native human PP.

### Human Y receptor-4 displacement assay:

The principle of the displacement assay is a fixed quantity of radiolabeled ligand competes with varying doses of a 'cold' (non-labelled) ligand for a limited number of receptor binding sites, enabling quantification of the affinity of the cold ligand to the receptor.

The human Y4 receptor was purchased from the UMR cDNA Resource Centre (catalogue number NPYR400000). The cDNA clone was transformed into competent *E.coli* so a larger preparation of DNA could be produced which was transfected into HEK-293T cells. Using antibiotics, cells expressing the transfected cDNA were selected for, and those cells containing the receptor plasmid were scaled up for a cell membrane preparation. Cells were harvested from culture flasks by scraping, and then homogenised and the membrane separated from other cellular material by differential centrifugation. The cell membrane was resuspended in a weak HEPES buffer containing protease inhibitors, before been aliquoted and the preparation stored at -70°C until use.

For the displacement assay all reagents are prepared in displacement assay buffer (25 mM HEPES pH 7.4, 2 mM MgCl₂; 4mM CaCl₂ 1% BSA, 4 mM) containing protease inhibitors (2 mM phenylmethanesulphonylfluoride, 0.5 mM diprotin A and 0.5 mM phosphoramidon). One thousand counts/sec/tube ¹¹²⁵PP (iodinated by the direct iodogen method) is used as the radiolabeled peptide, and the competing peptide is tested at varying concentrations between 10 uM and 0.02 pM, with each concentration tested in a minimum of duplicate. To each assay tube a volume of the prepared membrane, enough for total counts (where no competing peptide is present) of 10000 ―15000 counts/240 second is added. Reagents are added in the following order: buffer, label competing peptide and cell membrane, with a total volume is 500 µl.

The assay is incubated at room temperature for 90 minutes before the receptor-¹²⁵PP complex is separated from free label by centrifugation at 15600g for 3 minutes. The supernatant is removed and discarded and the pellet resuspended and washed with 500 µl fresh assay buffer and re-centrifuged. After centrifugation and removal of supernatant the radioactivity of the pellet was measured for 240 seconds using a γ-counter. Specific binding is calculated as the difference between the amount of ¹²⁵I-PP bound in the absence (total) and presence (non-specific) of unlabelled peptide. From the binding data, an IC₅₀ for the competing peptide is calculated. Each peptide is tested on a minimum of two separate occasions.

### In vivo food intake experiments

Male C57BL/6 mice (20-30g) were used for all mouse experiments. Mice were individually housed in IVC cages. Animals were randomised by weight and fasted overnight for 16hrs before intraperitoneal injection at 9am. All peptide solutions were prepared freshly the morning of the study and injected as 100ul. Food intake was measured at 1, 2, 3, 4, 6, 8 and 24 hours post injection. The peptide was dosed at 30mnol/kg, 50nmol/kg, 100nmol/kg, 120nmo1/kg, 150nmol/kg, 300nmol/kg, 600nmol/kg or 1000nmol/kg depending on the activity. In each case, a control of saline and a control of unmodified human Pancreatic Polypeptide was carried out. All statistics are calculated using a one-way ANOVA with Dunnett's post-test or one-way ANOVA with Bonferroni post-test.

### Results:

### A: Human Y receptor-4 binding

Native human Pancreatic Polypeptide was found to bind the Y4 receptor with an IC₅₀ of 0.23nM. In the Table in Figure 1 there is given under "Y4" the IC₅₀ of the analogues in nM. A number lower than 0.23 nM represents stronger binding than native human PP.

As is seen in the data in the Table in Figure 1, many of the compounds of the invention have a stronger binding affinity to the receptor than native human PP.

### B: Food intake reduction in mice

The effects of the PP analogues were compared with native human PP after a single injection. A control of saline was used.

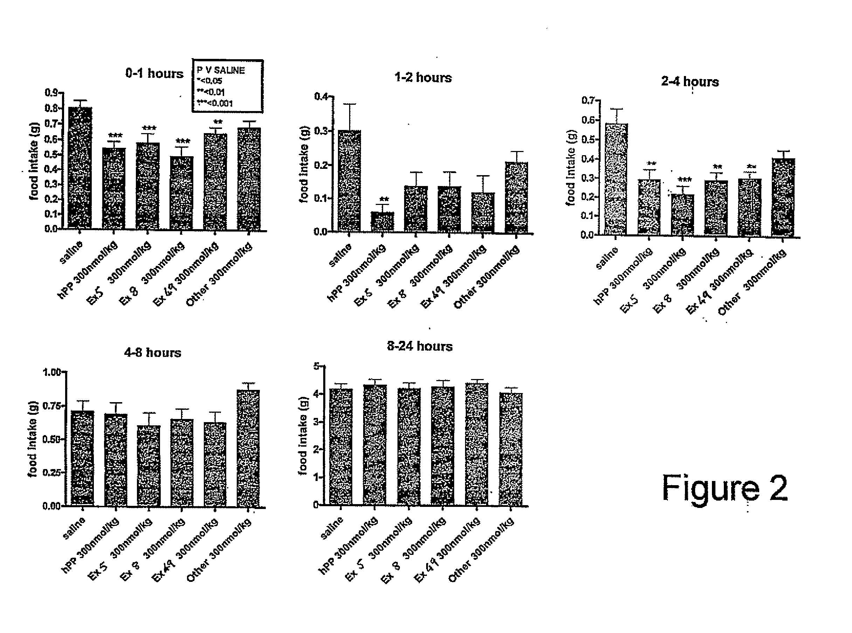

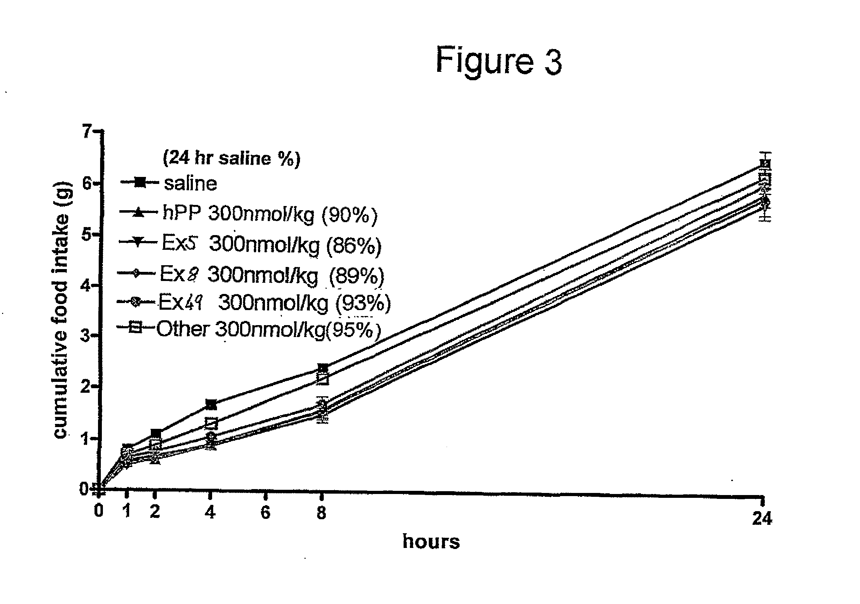

The detailed results for Examples 5, 8 and 49 are shown in the time-interval bar charts of Figure 2 and in the comparative graph of Figure 3. For the remaining compounds, the feeding data are shown in the Table in Figure 1. The column entitled "4-8 hours" is the reduction in food consumed between hour 4 and hour 8 as compared with saline expressed as the relative increase in food intake reduction as compared with native human PP. The column entitled "0-24 hours" is similarly the reduction in food consumed between hour 0 and hour 24 compared with saline control expressed as the relative increase in food intake reduction as compared with native human PP. For Example, if the same dose of an analogue resulted in the same reduction in feeding as the native PP control the entry in the columns would be 1.0. If the same dose of an analogue resulted in a greater reduction in feeding than the native PP control, the entry in the columns would be greater than 1. A result of greater than 1 means that total food intake over the period was lower with the analogue than the native human PP. It is hypothesized that that is as a possible result of stronger activity at the receptor and longer activity.

The column entitled "4:1" is the ratio between (the feeding reduction observed over the first 4 hours expressed as the relative increase in food intake reduction as compared with native human PP over the same time period) and (the feeding reduction observed over the first 1 hour expressed as the relative increase in food intake reduction as compared with native human PP over the same time period). Similarly, the column entitled "24:1" is the ratio between (the feeding reduction observed over the first 24 hours expressed as the relative increase in food intake reduction as compared with native human PP over the same time period) and (the feeding reduction observed over the first 1 hour expressed as the relative increase in food intake reduction as compared with native human PP over the same time period). A "4:1" or "24:1" ratio of greater than 1 indicates that the tail off in activity in time for the peptide in the mouse was slower than the tail off in activity of native human PP in the mouse, thus indicating that the peptide is longer lasting than native human PP, independent of the level of activity at the receptor. The mouse is a good model for degradation rates in humans, irrespective of the fact that the receptors for PP in mice are different from the human receptor.

Further features of this invention are described by reference to the following clauses:
1. An analogue of human Pancreatic Polypeptide (SEQ. ID No 1) which differs from native human pancreatic polypeptide in one of more of the following respects:

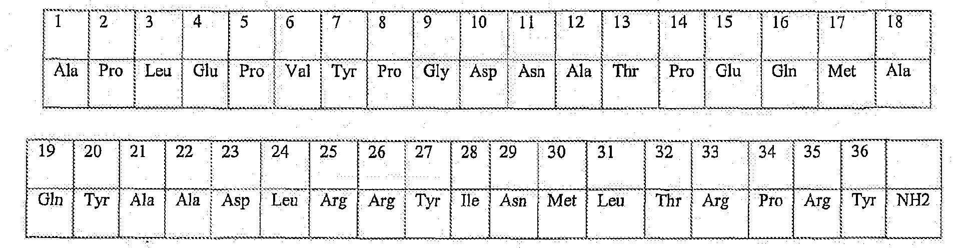

a) it has the Ala at position 1
(i) replaced by an alternative amino acid selected from D-Ala, Asp, Glu, Gly, His, Tyr, Lys, acylated Ala and acylated Lys, the acyl group being selected from: CO― C₁₋₂₀ alkyl, CO-C₂₋₂₀ alkenyl, CO-C₅₋₁₀ aryl and CO-C₅₋₁₀ ar-C₁₋₂₀ alkyl, and/or
(ii) furnished with an additional amino acid at position 0, selected from Gly, Lys, His, Glu, Asp, Pro, Ile, Phe, Val, Cys, Tyr, TyrTyrTyr, Ala, D-Ala, and acylated Ala, the acyl group being selected from: CO-C₁₋₂₀ alkyl, CO-C₂₋₂₀ alkenyl, CO-C₅₋₁₀ aryl and CO-C₅₋₁₀ ar-C₁₋₂₀ alkyl; or
(iii) absent
b) at positions 3, 4, 5, 6, 7 and 10, it has
(i) the Leu at position 3 replaced by the alternative amino acid Ile or Ser; and/or
(ii) the Glu at position 4 replaced by the alternative amino acid Lys; and/or
(iii) the Pro at position 5 replaced by the alternative amino acid Ala; and/or
(iv) the Val at position 6 replaced by an alternative amino acid selected from Glu, His, Ile, Leu, Ser, Phe, Cys, Thr, Ala, Arg, Asp, Lys, Tyr and Met; and/or
(v) the Tyr at position 7 replaced by the alternative amino acid Ala, Asn or Phe; and/or
(vi) the Asp at position 10 replaced by the alternative amino acid Glu;
c) it has the Asn at position 11 replaced by an alternative amino acid selected from Asp and Tyr; and/or the Thr at position 13 replaced by the alternative amino acid Ser;
d) at positions 14 to 26, it has one or more of the native amino acids 14 to 26 (first row, bold) replaced with an amino acid selected from the amino acids in the respective column below it:

| 14 | 15 | 16 | 17 | 18 | 19 | 20 | 21 | 22 | 23 | 24 | 25 | 26 |
|---|---|---|---|---|---|---|---|---|---|---|---|---|
| **Pro** | **Glu** | **Gln** | **Met** | **Ala** | **Gln** | **Tyr** | **Ala** | **Ala** | **Asp** | **Leu** | **Arg** | **Arg** |
| Glu | Gln | Glu | Leu | Asn | Arg | | Tyr | Ser | Ala | Met | Lys | His |
| | | Asp | NLeu | Val | Lys | | Phe | Ile | Glu | Cys | NLeu | Lys |
| | | Lys-Acyl* | Ile | Ser | His | | Glu | Val | Ser | NLeu | | |
| | | | Arg | | Glu | | | Thr | Gly | | | |
| | | | Thr | | | | | | Gln | | | |
| | | | Gln | | | | | | Ile | | | |
| | | | Glu | | | | | | Leu | | | |
| | | | Lys | | | | | | NLeu | | | |
| | | | Val | | | | | | Val | | | |

| | | | | | | | | | | | | |
|---|---|---|---|---|---|---|---|---|---|---|---|---|
| * the acyl group being selected from: CO-C₁₋₂₀ alkyl, CO-C₂₋₂₀ alkenyl, CO-C₅₋₁₀ aryl and CO-C₅₋₁₀ ar-C₁₋₂₀ alkyl | | | | | | | | | | | | |

e) at positions 29, 30 and 31, it has:
(i) the Met at position 30 replaced by an alternative amino acid selected from Leu, Arg, Glu, His, NLeu, Ile, Val, Phe, Thr, Asn, Cys and Lys; and/or
(ii) the Leu at position 31 replaced by an alternative amino acid selected from Ile and Val; and/or
(iii) the Asn at position 29 replaced by Asp
f) at positions 34, 35 and 36, it has:
(i) the Pro at position 34 replaced by the alternative amino acid Gln, Asn, DPro, Leu, His or NVa (Nor-valine); and/or
(ii) the Arg at position 35 replaced by the alternative amino acid Gln or HArg; and/or
(iii) the Tyr at position 36 replaced by the alternative amino acid Phe
g) it is dimerised by disulphide bond formation between a pair of Cys residues added to the two units at the N termini or as replacements for Leu at position 31 in the two units,
h) it is cyclised by disulphide bond formation between a pair of Cys residues added as a replacement amino acid residue at a pair of positions selected from the following list: 8 & 17, 8 & 20, 5 & 24, 2 & 27, or between a Cys residue added at a replacement amino acid at position 28 or 29 and a Cys residue added as an additional amino acid at position 0; whereby when the analogue has Ala1 absent then it has at least one further change other than one or more selected from Nle17 and Nle30,
whereby when the analogue has one or more of Nle17, Nle30 and His34, then it has at least one further change other than the one or more selected from Nle17, Nle30 and His34, and
whereby when the analogue has Gln at position 34, then it has at least one further change other than one or more selected from Ile31, Val31; ,Leu30 and NLeu30.
for use (i) in the treatment (including prophylactic treatment) of obesity or diabetes in a subject, or (ii) in the reduction of appetite in a subject, the reduction of food intake in a subject, or in the reduction of calorie intake in a subject.
2. A compound that is an analogue (II) of human Pancreatic Polypeptide (SEQ. ID No 1) which differs from native human pancreatic polypeptide in one of more of the following respects:

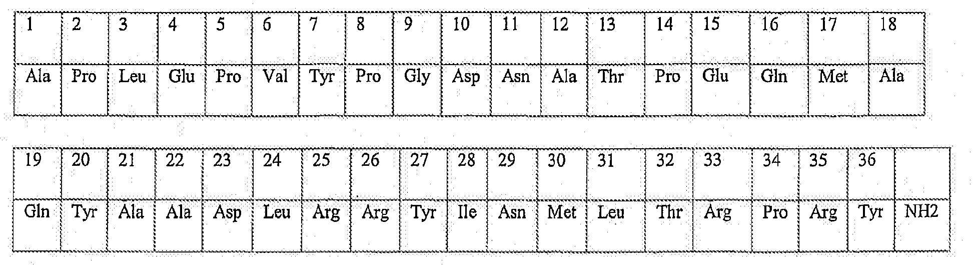

a) it has the Ala at position 1
(i) replaced by an alternative amino acid selected from D-Ala, Asp, Glu, Gly, His, Tyr, Lys, acylated Ala and acylated Lys, the acyl group being selected from: CO-C₁₋₂₀ alkyl, CO-C₂₋₂₀ alkenyl, CO-C₅₋₁₀ aryl and CO-C₅₋₁₀ ar: C₁₋₂₀ alkyl, and/or
(ii) furnished with an additional amino acid at position 0, selected from Gly, Lys, His, Glu, Asp, Pro, Ile, Phe, Val, Cys, Tyr, TyrTyrTyr, Ala, D-Ala, and acylated Ala, the acyl group being selected from: CO-C₁₋₂₀ alkyl, CO-C₂₋₂₀ alkenyl, CO-C₅-₁₀ acryl and CO-C₅₋₁₀ ar-C₁₋₂₀ alkyl; or
(iii) absent
b) at positions 3, 4, 5, 6, 7 and 10, it has
(i) the Leu at position 3 replaced by the alternative amino acid Ile or Ser; and/or
(ii) the Glu at position 4 replaced by the alternative amino acid Lys; and/or
(iii) the Pro at position 5 replaced by the alternative amino acid Ala; and/or
(iv) the Val at position 6 replaced by an alternative amino acid selected from Glu, His, Ile, Leu, Ser, Phe, Cys, Thr, Ala, Arg, Asp, Lys, Tyr and Met; and/or
(v) the Tyr at position 7 replaced by the alternative amino acid Ala, Asn or Phe; and/or
(vi) the Asp at position 10 replaced by the alternative amino acid Glu;
c) it has the Asn at position 11 replaced by an alternative amino acid selected from Asp and Tyr; and/or the Thr at position 13 replaced by the alternative amino acid Ser;
d) at positions 14 to 26, it has one or more of the native amino acids 14 to 26 (first row, bold) replaced with an amino acid selected from the amino acids in the respective column below it:

| 14 | 15 | 16 | 17 | 18 | 19 | 20 | 21 | 22 | 23 | 24 | 25 | 26 |
|---|---|---|---|---|---|---|---|---|---|---|---|---|
| **Pro** | **Glu** | **Gln** | **Met** | **Ala** | **Gln** | **Tyr** | **Ala** | **Ala** | **Asp** | **Leu** | **Arg** | **Arg** |
| Glu | Gln | Glu | Leu | Asn | Arg | | Tyr | Ser | Ala | Met | Lys | His |
| | | Asp | NLeu | Val | Lys | | Phe | Ile | Glu | Cys | NLeu | Lys |
| | | Lys-Acyl* | Ile | Ser | His | | Glu | Val | Ser | NLeu | | |
| | | | Arg | | Glu | | | Thr | Gly | | | |
| | | | Thr | | | | | | Gln | | | |
| | | | Gln | | | | | | Ile | | | |
| | | | Glu | | | | | | Leu | | | |
| | | | Lys | | | | | | NLeu | | | |
| | | | Val | | | | | | Val | | | |

| | | | | | | | | | | | | |
|---|---|---|---|---|---|---|---|---|---|---|---|---|
| * the acyl group being selected from: CO-C₁₋₂₀ alkyl, CO-C₂₋₂₀ alkenyl, CO-C₅₋₁₀ aryl and CO-C₅₋₁₀ ar-C₁₋₂₀ alkyl | | | | | | | | | | | | |

e) at positions 29, 30 and 31, it has:
(i) the Met at position 30 replaced by an alternative amino acid selected from Leu, Arg, Glu, His, NLeu, Ile, Val, Phe, Thr, Asn, Cys and Lys; and/or
(ii) the Leu at position 31 replaced by an alternative amino acid selected from Ile and Val; and/or
(iii) the Asn at position 29 replaced by Asp
f) at positions 34, 35 and 36, it has:
(i) the Pro at position 34 replaced by the alternative amino acid Gln, Asn, DPro, Leu, His or NVa (Nor-valine); and/or
(ii) the Arg at position 35 replaced by the alternative amino acid Gln or HArg; and/or
(iii) the Tyr at position 36 replaced by the alternative amino acid Phe
g) it is dimerised by disulphide bond formation between a pair of Cys residues added to the two units at the C termini or as replacements for Leu at position 31 in the two units,
h) it is cyclised by disulphide bond formation between a pair of Cys residues added as a replacement amino acid residue at a pair of positions selected from the following list: 8 & 17, 8 & 20, 5 & 24, 2 & 27, or between a Cys residue added at a replacement amino acid at position 28 or 29 and a Cys residue added as an additional amino acid at position 0; whereby when the analogue has Ala1 absent then it has at least one further change other than one or more selected from Nle17 and Nle30,
whereby when the analogue has one or more of Nle17, Nle30 and His34, then it has at least one further change other than the one or more selected from Nle17, N1e30 and His34, and
whereby when the analogue has Gln at position 34, then it has at least one further change other than one or more selected from Ile31, Val31, Leu30 and NLeu30.
or a variant or derivative thereof; or a salt or solvate thereof,
and whereby if the analogue has one or more of:
- Arg at position 19 (optionally with Phe at position 6)
- Tyr or Glu at position 21 (optionally with Phe at position 6)
- Ser at position 22 (optionally with Phe at position 6)
- Ala or Gln at position 23 (optionally with Phe at position 6)
or if the analogue has one of:
- Glu at position 23 and Glu at position 6
- Asp at position 11 and Gln at position 15 and Glu at position 23 and Met at position 24
- Met at position 6 and Tyr at position 11 and Glu at position 21 and Thr at position 22 and
- Gln at position 23 and Thr at position 30
then the analogue has at least one further difference from the native human Pancreatic Polypeptide.
3. A compound as claimed in clause1 or clause 2, having the amino acid sequence
Xaa0-Xaa1-Pro-Leu-Glu-Pro-Xaa6-Xaa7-Pro-Gly-Xaa10-Xaa11-Ala-Xaa13-Pro-Xaa15-Xaa16-Xaa17-Xaa18-Xaa19-Tyr-xaa21-Xaa22-Xaa23-Leu-Arg-Arg-Tyr-Ile-Xaa29-Xaa30-Leu-Thr-Arg-Xaa34-Arg-Xaa36-NH2,
Wherein: Xaa0-Xaa1 is Pro-Ala, Gly-Ala, His-Ala, Ala-Ala, Tyr-Ala, Ala or absent;
Xaa6 is Glu, Ser, Thr, Val, His, Lys, His, Ile or Asp;
Xaa7 is Tyr or Ala;
Xaa10 'is Asp or Glu;
Xaa11 is Asn or Asp;
Xaa13 is Thr or Ser;
Xaa15 is Glu or Gln;
Xaa26 is Glu, Gln or Asp;
Xaa17 is Met, Leu, Lys or Ile
Xaa18 is Ala or Asn;
Xaa19 is Arg, Lys or His;
Xaa 21 is Ala or Tyr;
Xaa 22 isAla or Ser;
Xaa 23 is Glu, Ala or Asp;
Xaa 29 is Asp or Asn;
Xaa 3 0 is Lys, His or Arg;
Xaa 34 is N-Val or Pro: and
Xaa 36 is Tyr or Phe.
4. A compound as claimed in one of clauses1 to 3 in which the variant has additional 1, 2, 3 or 4 modifications selected from deletions, insertions, inversions, repeats and substitutions.
5. A compound as claimed in any one clause 1 to 4 which includes at least 30 amino acids of the native human PP sequence.
6. A compound as claimed in clause 1or clause 2is a compound of any one of Examples 2 to 1206.
7. A compound as claimed in clause 1or clause 2 having the sequence
Gly Ala Pro Leu Glu Pro Thr Tyr Pro Gly Asp Asn Ala Thr Pro Glu Gln Leu Ala Lys Tyr Tyr Ser Glu Leu Arg Arg Tyr Ile Asn Lys Leu Thr Arg Pro Arg Tyr NH2; or
Gly Ala Pro Leu Glu Pro Glu Tyr Pro Gly Asp Asn Ala Thr Pro Glu Gln Met Ala Arg Tyr Ala Ala Ala Leu Arg Arg Tyr Ile Asn Lys Leu Thr Arg Pro Arg Tyr NH2),
or a derivative thereof.
8. A compound as claimed in any one of clause 1 to 7 which is a derivative that is modified by one or more processes selected from amidation, glycosylation, carbamylation, sulfation, phosphylation, cyclization, lipidization and pegylation.
9. A compound as claimed in any one of clauses 1 to 8 which is a derivative that is a fusion protein.
10. A pharmaceutical composition comprising a compound as claimed in any one of
11. A method of treating obesity or diabetes in a subject in need thereof comprising administering to the subj ect a compound as claimed in any one of clause 1 to 9 or a composition as claimed in clause 10
12. A method of reducing appetite in a subject, reducing food intake in a subject, or reducing calorie intake in a subject, comprising administering to the subject a compound as claimed in any one of clauses 1 to 9 or a composition as claimed in clause 10
13. The method as claimed in clause 11 or clause 12, wherein the subject is overweight.
14. The method as claimed in clause 11or clause12, wherein the subject is obese.
15. The method as claimed in clause 11or clause 12, wherein the subject is diabetic.
16. The method as claimed in any one of clauses 11 to 15, wherein the compound is administered peripherally.
17. The method as claimed in any one of clauses 11 to 16 wherein the compound is administered subcutaneously, intravenously, intramuscularly, intranasally, transdermally or sublingually.
18. Use of a compound as claimed in any one of clauses 1 to 9 for the manufacture of a medicament for the treatment (including prophylactic treatment) of obesity or diabetes.
19. Use of a compound as claimed in any one of clauses 1 to 9 for the manufacture of a medicament for the reduction of appetite in a subject, for the reduction of food intake in a subject, or for the reduction of calorie intake in a subject.
20. A method of cosmetic weight loss, comprising administering to the subject a compound as claimed in any one of clauses 1 to 10 or a composition as claimed in clause 11.
21. The method as claimed in clause 20, wherein the compound is administered peripherally.
22. The method as claimed in clause 20, wherein the compound is administered subcutaneously, intravenously, intramuscularly, intranasally, transdermally or sublingually.

## Claims

1. An analogue of human Pancreatic Polypeptide (SEQ. ID No 1) which differs from native human pancreatic polypeptide in that: 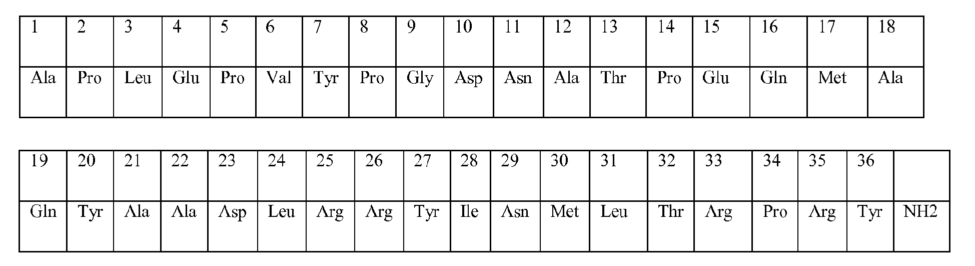
a) it has the Ala at position 1 furnished with an additional amino acid at position 0, selected from Gly, Lys, His, Glu, Asp, Pro, Ile, Phe, Val, Cys, Tyr, TyrTyrTyr, Ala, D-Ala, and acylated Ala, the acyl group being selected from: CO-C₁₋₂₀ alkyl, CO-C₂₋₂₀ alkenyl, CO-C₅₋₁₀ aryl and CO-C₅₋₁₀ ar-C₁₋₂₀ alkyl;
and which may further differ from native human pancreatic polypeptide in one or more of the following respects:
b) at positions 3, 4, 5, 6, 7 and 10, it has
(i) the Leu at position 3 replaced by the alternative amino acid Ile or Ser; and/or
(ii) the Glu at position 4 replaced by the alternative amino acid Lys; and/or
(iii) the Pro at position 5 replaced by the alternative amino acid Ala; and/or
(iv) the Val at position 6 replaced by an alternative amino acid selected from Glu, His, Ile, Leu, Ser, Phe, Cys, Thr, Ala, Arg, Asp, Lys, Tyr and Met; and/or
(v) the Tyr at position 7 replaced by the alternative amino acid Ala, Asn or Phe; and/or
(vi) the Asp at position 10 replaced by the alternative amino acid Glu;
c) it has the Asn at position 11 replaced by an alternative amino acid selected from Asp and Tyr; and/or the Thr at position 13 replaced by the alternative amino acid Ser;
d) at positions 14 to 26, it has one or more of the native amino acids 14 to 26 (first row, bold) replaced with an amino acid selected from the amino acids in the respective column below it:
| 14 | 15 | 16 | 17 | 18 | 19 | 20 | 21 | 22 | 23 | 24 | 25 | 26 |
|---|---|---|---|---|---|---|---|---|---|---|---|---|
| **Pro** | **Glu** | **Gln** | **Met** | **Ala** | **Gln** | **Tyr** | **Ala** | **Ala** | **Asp** | **Leu** | **Arg** | **Arg** |
| Glu | Gln | Glu | Leu | Asn | Arg | | Tyr | Ser | Ala | Met | Lys | His |
| | | Asp | NLeu | Val | Lys | | Phe | Ile | Glu | Cys | NLeu | Lys |
| | | Lys-Acyl* | Ile | Ser | His | | Glu | Val | Ser | NLeu | | |
| | | | Arg | | Glu | | | Thr | Gly | | | |
| | | | Thr | | | | | | Gln | | | |
| | | | Gln | | | | | | Ile | | | |
| | | | Glu | | | | | | Leu | | | |
| | | | Lys | | | | | | NLeu | | | |
| | | | Val | | | | | | Val | | | |
| | | | | | | | | | | | | |
|---|---|---|---|---|---|---|---|---|---|---|---|---|
| * the acyl group being selected from: CO-C₁₋₂₀ alkyl, CO-C₂₋₂₀ alkenyl, CO-C₅₋₁₀ aryl and CO-C₅₋₁₀ ar-C₁₋₂₀ alkyl | | | | | | | | | | | | |
e) at positions 29, 30 and 31, it has:
(i) the Met at position 30 replaced by an alternative amino acid selected from Leu, Arg, Glu, His, NLeu, Ile, Val, Phe, Thr, Asn, Cys and Lys; and/or
(ii) the Leu at position 31 replaced by an alternative amino acid selected from Ile and Val; and/or
(iii) the Asn at position 29 replaced by Asp
f) at positions 34, 35 and 36, it has:
(i) the Pro at position 34 replaced by the alternative amino acid Gln, Asn, DPro, Leu, His or NVa (Nor-valine); and/or
(ii) the Arg at position 35 replaced by the alternative amino acid Gln or HArg; and/or
(iii) the Tyr at position 36 replaced by the alternative amino acid Phe
g) it is dimerised by disulphide bond formation between a pair of Cys residues added to the two units at the N termini or as replacements for Leu at position 31 in the two units,
h) it is cyclised by disulphide bond formation between a pair of Cys residues added as a replacement amino acid residue at a pair of positions selected from the following list: 8 & 17, 8 & 20, 5 & 24, 2 & 27, or between a Cys residue added at a replacement amino acid at position 28 or 29 and a Cys residue added as an additional amino acid at position 0;
whereby when the analogue has one or more of Nle17, Nle30 and His34, then it has at least one further change other than the one or more selected from Nle17, Nle30 and His34, and
whereby when the analogue has Gln at position 34, then it has at least one further change other than one or more selected from Ile31, Val31, Leu30 and NLeu30.
for use (i) in the treatment (including prophylactic treatment) of obesity or diabetes in a subject, or (ii) in the reduction of appetite in a subject, the reduction of food intake in a subject, or in the reduction of calorie intake in a subject.

2. A compound that is an analogue (II) of human Pancreatic Polypeptide (SEQ. ID No 1) which differs from native human pancreatic polypeptide in one of more of the following respects: 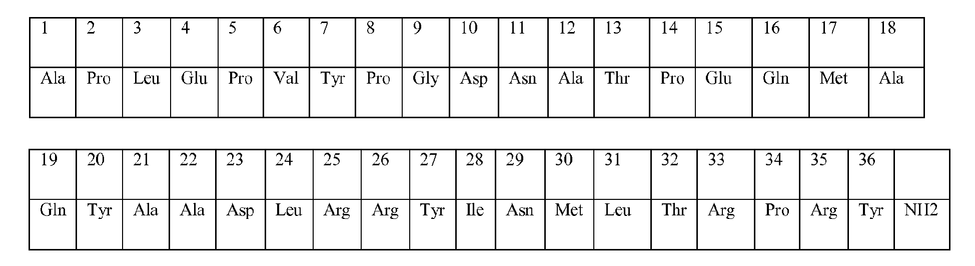
a) it has the Ala at position 1
furnished with an additional amino acid at position 0, selected from Gly, Lys, His, Glu, Asp, Pro, Ile, Phe, Val, Cys, Tyr, TyrTyrTyr, Ala, D-Ala, and acylated Ala, the acyl group being selected from: CO-C₁₋₂₀ alkyl, CO-C₂₋₂₀ alkenyl, CO-C₅₋₁₀ aryl and CO-C₅₋₁₀ ar-C₁₋₂₀ alkyl; and which may further differ from native human pancreatic polypeptide in one or more of the following respects:
b) at positions 3, 4, 5, 6, 7 and 10, it has
(i) the Leu at position 3 replaced by the alternative amino acid Ile or Ser; and/or
(ii) the Glu at position 4 replaced by the alternative amino acid Lys; and/or
(iii) the Pro at position 5 replaced by the alternative amino acid Ala; and/or
(iv) the Val at position 6 replaced by an alternative amino acid selected from Glu, His, Ile, Leu, Ser, Phe, Cys, Thr, Ala, Arg, Asp, Lys, Tyr and Met; and/or
(v) the Tyr at position 7 replaced by the alternative amino acid Ala, Asn or Phe; and/or
(vi) the Asp at position 10 replaced by the alternative amino acid Glu;
c) it has the Asn at position 11 replaced by an alternative amino acid selected from Asp and Tyr; and/or the Thr at position 13 replaced by the alternative amino acid Ser;
d) at positions 14 to 26, it has one or more of the native amino acids 14 to 26 (first row, bold) replaced with an amino acid selected from the amino acids in the respective column below it:
| 14 | 15 | 16 | 17 | 18 | 19 | 20 | 21 | 22 | 23 | 24 | 25 | 26 |
|---|---|---|---|---|---|---|---|---|---|---|---|---|
| **Pro** | **Glu** | **Gln** | **Met** | **Ala** | **Gln** | **Tyr** | **Ala** | **Ala** | **Asp** | **Leu** | **Arg** | **Arg** |
| Glu | Gln | Glu | Leu | Asn | Arg | | Tyr | Ser | Ala | Met | Lys | His |
| | | Asp | NLeu | Val | Lys | | Phe | Ile | Glu | Cys | NLeu | Lys |
| | | Lys-Acyl* | Ile | Ser | His | | Glu | Val | Ser | NLeu | | |
| | | | Arg | | Glu | | | Thr | Gly | | | |
| | | | Thr | | | | | | Gln | | | |
| | | | Gln | | | | | | Ile | | | |
| | | | Glu | | | | | | Leu | | | |
| | | | Lys | | | | | | NLeu | | | |
| | | | Val | | | | | | Val | | | |
| | | | | | | | | | | | | |
|---|---|---|---|---|---|---|---|---|---|---|---|---|
| * the acyl group being selected from: CO-C₁₋₂₀ alkyl, CO-C₂₋₂₀ alkenyl, CO-C₅₋₁₀ aryl and CO―C₅₋₁₀ ar―C₁₋₂₀ alkyl | | | | | | | | | | | | |
e) at positions 29, 30 and 31, it has:
(i) the Met at position 30 replaced by an alternative amino acid selected from Leu, Arg, Glu, His, NLeu, Ile, Val, Phe, Thr, Asn, Cys and Lys; and/or
(ii) the Leu at position 31 replaced by an alternative amino acid selected from Ile and Val; and/or
(iii) the Asn at position 29 replaced by Asp
f) at positions 34, 35 and 36, it has:
(i) the Pro at position 34 replaced by the alternative amino acid Gln, Asn, DPro, Leu, His or NVa (Nor-valine); and/or
(ii) the Arg at position 35 replaced by the alternative amino acid Gln or HArg; and/or
(iii) the Tyr at position 36 replaced by the alternative amino acid Phe
g) it is dimerised by disulphide bond formation between a pair of Cys residues added to the two units at the C termini or as replacements for Leu at position 31 in the two units,
h) it is cyclised by disulphide bond formation between a pair of Cys residues added as a replacement amino acid residue at a pair of positions selected from the following list: 8 & 17, 8 & 20, 5 & 24, 2 & 27, or between a Cys residue added at a replacement amino acid at position 28 or 29 and a Cys residue added as an additional amino acid at position 0;
whereby when the analogue has one or more of Nle17, Nle30 and His34, then it has at least one further change other than the one or more selected from Nle17, Nle30 and His34, and
whereby when the analogue has Gln at position 34, then it has at least one further change other than one or more selected from Ile31, Val31, Leu30 and NLeu30.
or a variant or derivative thereof; or a salt or solvate thereof,
and whereby if the analogue has one or more of:
- Arg at position 19 (optionally with Phe at position 6)
- Tyr or Glu at position 21 (optionally with Phe at position 6)
- Ser at position 22 (optionally with Phe at position 6)
- Ala or Gln at position 23 (optionally with Phe at position 6)
or if the analogue has one of:
- Glu at position 23 and Glu at position 6
- Asp at position 11 and Gln at position 15 and Glu at position 23 and Met at position 24
- Met at position 6 and Tyr at position 11 and Glu at position 21 and Thr at position 22 and
- Gln at position 23 and Thr at position 30
then the analogue has at least one further difference from the native human Pancreatic Polypeptide.

3. A compound as claimed in claim 1 or claim 2, having the amino acid sequence
Xaa0-Xaa1-Pro-Leu-Glu-Pro-Xaa6-Xaa7-Pro-Gly-Xaa10-Xaa11-Ala-Xaa13-Pro-Xaa15-Xaa16-Xaa17-Xaa18-Xaa19-Tyr-xaa21-Xaa22-Xaa23-Leu-Arg-Arg-Tyr-He-Xaa29-Xaa30-Leu-Thr-Arg-Xaa34-Arg-Xaa36-NH2,
Wherein: Xaa0-Xaa1 is Pro-Ala, Gly-Ala, His-Ala, Ala-Ala, Tyr-Ala;
Xaa6 is Glu, Ser, Thr, Val, His, Lys, His, Ile or Asp;
Xaa7 is Tyr or Ala;
Xaa10 is Asp or Glu;
Xaa11 is Asn or Asp;
Xaa13 is Thr or Ser;
Xaa15 is Glu or Gln;
Xaa16 is Glu, Gln or Asp;
Xaa17 is Met, Leu, Lys or Ile
Xaa18 is Ala or Asn;
Xaa19 is Arg, Lys or His;
Xaa 21 is Ala or Tyr;
Xaa 22 is Ala or Ser;
Xaa 23 isGlu, Ala or Asp;
Xaa 29 is Asp or Asn;
Xaa 30 is Lys, His or Arg;
Xaa 34 is N-Val or Pro: and
Xaa 36 is Tyr or Phe.

4. A compound as claimed in one of claims 1 to 3 in which the variant has additional 1, 2, 3 or 4 modifications selected from deletions, insertions, inversions, repeats and substitutions.

5. A compound as claimed in any one of claims 1 to 4 which includes at least 30 amino acids of the native human PP sequence.

6. A compound as claimed in claim 1 or claim 2 which is a compound of one of Examples 2 to 1206.

7. A compound as claimed in claim 1 or claim 2 having the sequence
Gly Ala Pro Leu Glu Pro Thr Tyr Pro Gly Asp Asn Ala Thr Pro Glu Gln Leu Ala Lys Tyr Tyr Ser Glu Leu Arg Arg Tyr Ile Asn Lys Leu Thr Arg Pro Arg Tyr NH2; or
Gly Ala Pro Leu Glu Pro Glu Tyr Pro Gly Asp Asn Ala Thr Pro Glu Gln Met Ala Arg Tyr Ala Ala Ala Leu Arg Arg Tyr Ile Asn Lys Leu Thr Arg Pro Arg Tyr NH2),
or a derivative thereof.

8. A compound as claimed in any one of claims 1 to 7 which is a derivative that is modified by one or more processes selected from amidation, glycosylation, carbamylation, sulfation, phosphylation, cyclization, lipidization and pegylation.

9. A compound as claimed in any one of claims 1 to 8 which is a derivative that is a fusion protein.

10. A pharmaceutical composition comprising a compound as claimed in any one of claims 1 to 9.

11. A method of treating obesity or diabetes in a subject in need thereof comprising administering to the subject a compound as claimed in any one of claims 1 to 9 or a composition as claimed in claim 10.

12. A method of reducing appetite in a subject, reducing food intake in a subject, or reducing calorie intake in a subject, comprising administering to the subject a compound as claimed in any one of claims 1 to 9 or a composition as claimed in claim 10.

13. The method as claimed in claim 11 or claim 12, wherein the subject is overweight.

14. The method as claimed in claim 11 or claim 12, wherein the subject is obese.

15. The method as claimed in claim 11 or claim 12, wherein the subject is diabetic.

16. The method as claimed in any one of claims 11 to 15, wherein the compound is administered peripherally.

17. The method as claimed in any one of claims 11 to 16 wherein the compound is administered subcutaneously, intravenously, intramuscularly, intranasally, transdermally or sublingually.

18. Use of a compound as claimed in any one of claims 1 to 9 for the manufacture of a medicament for the treatment (including prophylactic treatment) of obesity or diabetes.

19. Use of a compound as claimed in any one of claims 1 to 9 for the manufacture of a medicament for the reduction of appetite in a subject, for the reduction of food intake in a subject, or for the reduction of calorie intake in a subject.

20. A method of cosmetic weight loss, comprising administering to the subject a compound as claimed in any one of claims 1 to 10 or a composition as claimed in claim 11.

21. The method as claimed in claim 20, wherein the compound is administered peripherally.

22. The method as claimed in claim 20, wherein the compound is administered subcutaneously, intravenously, intramuscularly, intranasally, transdermally or sublingually.
